# EUROPEAN PATENT APPLICATION

(11) **EP 4 397 762 A1**
(43) Date of publication of application: **10.07.2024**
(21) Application number: 22864537.0
(22) Date of filing: 30.08.2022
(51) Int. Cl.: C12N 15/13, A61K 39/395, A61P 3/10, A61P 5/08, A61P 11/00, A61P 13/12, A61P 19/02, A61P 29/00, A61P 35/00, A61P 43/00, C07K 16/26, C12N 1/15, C12N 1/19, C12N 1/21, C12N 5/10, C12N 15/63, C12P 21/08

(54) **ANTI-GROWTH HORMONE ANTIBODY**

(30) Priority: 31.08.2021 JP 2021140932
(71) Applicant: Taisho Pharmaceutical Co., Ltd., Tokyo 170-8633 (JP)
(72) Inventor: KUROKAWA, Tomofumi, Tokyo 170-8633 (JP); UEMATSU, Yoshikatsu, Tokyo 170-8633 (JP); HATA, Tomoyuki, Tokyo 170-8633 (JP); ADACHI, Hisashi, Tokyo 170-8633 (JP); SUGITA, Ayumi, Tokyo 170-8633 (JP)
(74) Representative: Hoffmann Eitle
(86) International application number: PCT/JP2022/032523
(87) International publication number: WO 2023/032955

(57) **Abstract**

In one embodiment, the present invention provides an antibody specifically binding to human GH or an antigen-binding fragment thereof. In one embodiment, the present invention relates to an antibody specifically binding to human GH or an antigen-binding fragment thereof, comprising a heavy chain variable region (VH) and a light chain variable region (VL), wherein the VH comprises an amino acid sequence set forth in SEQ ID NO: 5 as a VH complementarity-determining region (CDR) 1 (VHCDR1), an amino acid sequence set forth in SEQ ID NO: 6 as a VHCDR2, and an amino acid sequence set forth in SEQ ID NO: 7 as a VHCDR3, and the VL comprises an amino acid sequence set forth in SEQ ID NO: 8 as a VL complementarity-determining region (CDR) 1 (VLCDR1), an amino acid sequence set forth in SEQ ID NO: 9 as a VLCDR2, and an amino acid sequence set forth in SEQ ID NO: 10 as a VLCDR3.

## Description

### Technical Field

In one embodiment, the present invention relates to an antibody specifically binding to human growth hormone or an antigen-binding fragment thereof, a nucleic acid encoding the same, a vector comprising the nucleic acid, a cell comprising the nucleic acid or vector, a medicine comprising the antibody or antigen-binding fragment thereof, or a method for decreasing the level of an insulin-like growth factor 1 (IGF-1) in the blood, comprising administration of a therapeutically effective amount of the antibody or antigen-binding fragment thereof.

### Background Art

Growth hormone (hereinafter, also referred to as "GH") is a proteinaceous hormone synthesized and secreted from the pituitary gland. GH isoforms having molecular weights of 22 kDa and 20 kDa (GH-22K and GH-20K) are known. While the major molecular species in human serum is GH-22K, other than this, the presence of several isoforms such as 20 kDa (GH-20K) is known (Non Patent Literature 1). In addition, in pregnant women, GH isoforms having molecular weights of 22 kDa and 20 kDa (GH-V-22K and GH-V-20K) are secreted from the placenta (Non Patent Literature 2). GH exhibits effects regarding growth such as elongation of bone and growth of muscle, and effects regarding metabolism such as metabolic promotion, increase in blood sugar level and maintenance of homeostasis, both by direct action on tissues and by stimulation of release of insulin-like growth factor-1 (hereinafter, also referred to "IGF-1") through acting on a growth hormone receptor (hereinafter, also referred to "GHR") that is expressed in the liver and other tissues (Non Patent Literature 3).

Hypersecretion and hyposecretion of GH are associated with numerous diseases. GH hypersecretion is caused by benign pituitary adenoma, and as the typical symptoms thereof, acromegaly and gigantism are known. Acromegaly is a disease also called "hypertrophy of extremities", and the tips of the body, such as the forehead, nose and chin, limbs, and tongue become enlarged in association with bristling body hair, hyperplasia of skin, excessive sweating and offensive body odor due to enlargement of the sebaceous and sweating glands, lowering voice pitch due to proliferation of cartilage in the oral cavity, and the like. When GH hypersecretion develops by puberty, it results in gigantism.

The prevalence of acromegaly has been reported to be between 4 and 24 people per 100,000 population, which is not high in number, and therefore, it is considered to be a rare disease. However, if GH hypersecretion lasts for a long time, symptoms such as metabolic abnormality, e.g., diabetes, hypertension, and hyperlipidemia, sleep apnea syndrome, or cardiac hypertrophy is associated in addition to the above symptoms, and further, there is a risk to cause angina pectoris, myocardial infarction, cerebrovascular disorder or the like. The mortality has been found to be higher if GH hypersecretion was overlooked and not treated. In addition, acromegaly is also known to increase the likelihood of complications such as colon cancer, thyroid cancer and the like, and therefore early detection and treatment are important.

As the diseases caused by GH hypersecretion, acromegaly and gigantism, as well as diabetic nephropathy, arthritis, pneumonia and the like are known. Non Patent Literature 4 discloses that GH expression by tumor cell is associated with the progression of endometrial cancers, breast cancers and the like; and Non Patent Literature 5 describes the tumor suppression effect of the GH receptor antagonist pegvisomant in tumor model mice. Non Patent Literature 6 describes involvement of GH with diabetic nephropathy, Non Patent Literature 7 describes involvement of GH in joint pain and arthritis, and Non Patent Literature 8 describes involvement of IGF-1 in pneumonia.

As the treatment methods for acromegaly and gigantism, removal and radiotherapy for regression of pituitary adenoma that excessively secretes GH, and medical treatment have been administered. The removal of pituitary adenoma has a risk of complication including death as in other surgeries, and it requires an advanced technique. The radiotherapy is also associated with a similar risk, and it may take several years to exert the effect. Currently, as commercially available therapeutic agents, there are somatostatin analogs (e.g., octreotide acetate sustained-release formulation, lanreotide acetate sustained-release formulation), dopamine agonists (bromocriptine mesylate), and GH-receptor antagonists (pegvisomant); however, agents satisfying efficacy, safety and convenience at the same time do not yet exist.

Patent Literature 1 discloses an anti-human GH mouse monoclonal antibody (mAb) (hGH-25 and hGH-26) binding to GH-22K but not substantially binding to GH-20K, an anti-human GH mouse mAb (hGH-33) binding to GH-20K but not substantially binding to GH-22K, and an anti-human GH mouse mAb (hGH-12) binding to both GH-22K and GH-20K. It has been shown that hGH-25 and hGH-26 inhibited only GH-22K-dependent cell proliferation, hGH-33 inhibited only GH-20K-dependent cell proliferation, and hGH-12 inhibited both GH-22K and GH-20K-dependent cell proliferation in the GH-dependent cell proliferation assay using Ba/F3 cell line forcibly expressing human GHR/G-CSFR. However, the strength of inhibition (IC₅₀: 50% inhibition concentration) for these antibodies were not clearly shown.

Non Patent Literature 9 discloses that eight anti-human GH mouse mAbs were obtained, and among them, clones EB1, EB2 and NA71 which are cross-reactive to human placental somatomammotropin (hCS) (human placental lactogen (hPL)), suppressed human GH-dependent and hCS-dependent proliferation of Nb₂ rat lymphoma cell line. However, the strength of inhibition (IC₅₀) for these antibodies were not clearly shown.

Non Patent Literature 10 discloses that the anti-human GH mouse mAb EB1 and EB2 described in Non Patent Literature 1 conversely promoted cartilage cell-proliferative effects in dwarf mice and mammary gland stimulating effect in pigeon of human GH and hCS.

Non Patent Literature 11 describes several anti-human GH mouse mAbs and shows that clones EB1 and EB2 described in Non Patent Literatures 10 and 11 inhibited binding of human GH to human GHR and inhibited inhibitory activity of human GH against glucose uptake into 3T3-F422A preadipocyte cells. However, the strength of inhibition (IC₅₀) for these antibodies were not clearly shown.

Non Patent Literature 12 is related to Patent Literature 1. Non Patent Literature 12 shows that hGH-25 and hGH-26 inhibited only GH-22K-dependent cell proliferation, hGH-33 inhibited only GH-20K-dependent cell proliferation, and hGH-12 inhibited both GH-22K and GH-20K-dependent cell proliferation in the GH-dependent cell proliferation assay using Ba/F3 cell line forcibly expressing human GHR/G-CSFR. However, the strength of inhibition (IC₅₀) for these antibodies were not clearly shown.

Non Patent Literature 13 describes three anti-human GH mouse mAbs and shows that, among of them, two clones (AC8 and F11) which strongly inhibit the binding of human GH to Nb₂ rat lymphoma cell line inhibited human GH-dependent Nb₂ cell proliferation. However, the strength of inhibition (IC₅₀) for these antibodies were not clearly shown.

Non Patent Literature 14 describes ten human GH-specific mouse mAbs, and shows that the antibodies suppressed T-cell proliferation induced by mitogen (PHA) stimulation. However, the inhibitory activity to human GH was not shown.

Non Patent Literature 15 describes three anti-human GH mouse mAbs and shows that only the clone (B-2) which is cross-reactive to hPL in high concentration inhibited human GH-human GHR binding. However, the strength of inhibition (IC₅₀) of the antibody was not clearly shown.

Non Patent Literature 16 describes a novel method for measuring human GH activity in serum using Ba/F3 cell line forcibly expressing human GHR (Ba/F3-hGHR), in which anti-human GH mouse mAb (clone 5801) was used as a positive control antibody to show the proliferation of Ba/F3-hGHR cells being GH-dependent. The 5801 antibody at 50-fold or 100-fold molar amount with respect to human GH completely inhibited the GH-dependent proliferation of Ba/F3-hGHR cells. However, the strength of inhibition (IC₅₀) for the antibody was not clearly shown.

Non Patent Literature 17 describes an anti-human GH mouse mAb (6J33) having neutralizing activity. Although it was shown that the 6J33 antibody had neutralizing activity, the strength of inhibition (IC₅₀) was not clearly shown.

### Citation List

### Patent Literature

Patent Literature 1: International Publication No. 1997/36929

### Non Patent Literature

Non Patent Literature 1:
   Growth hormone isoforms and segments/fragments: Molecular structure and laboratory measurement (Palo E. F. D. et al., Clinica Chimica Acta 2006, 364, 67-76)
Non Patent Literature 2:
   Cloning of two novel growth hormone transcripts expressed in human placenta (Boguszewski C. L. et al., J Clin Endocrinol Metab 1998, 83,2878-2885)
Non Patent Literature 3:
   Targeting growth hormone function: strategies and therapeutic applications (Lu M. et al., Signal Transduction and Targeted Therapy 2019, 4:3)
Non Patent Literature 4:
   Tumour-Derived Human Growth Hormone As a Therapeutic Target in Oncology (Jo K Perry et al., Trends Endocrinol Metab. 2017;28(8):587-596.)
Non Patent Literature 5:
   Antitumor activity of the growth hormone receptor antagonist pegvisomant against human meningiomas in nude mice (I E McCutcheon et al., J Neurosurg. 2001;94:487-492.)
Non Patent Literature 6:
   The glomerular podocyte as a target of growth hormone action: implications for the pathogenesis of diabetic nephropathy (P Anil Kumar et al., Curr Diabetes Rev. 2011;7 (1) :50-55.)
Non Patent Literature 7:
   Medical progress: Acromegaly (Shlomo Melmed, N Engl J Med 2006; 355:2558-2573)
Non Patent Literature 8:
   Adenovirus-mediated gene transfer of hIGF-IB in mouse lungs induced prolonged inflammation but no fibroproliferation (Caroline Leger et al., Am J Physiol Lung Cell Mol Physiol. 2010;298(4):L492-500.)
Non Patent Literature 9:
   Study of antigenic structure and inhibition of activity of human growth hormone and chorionic somatomammotropin by monoclonal antibodies. (Ivanyi J., Mol. Immunol. 19: 1611-1618, 1982.)
Non Patent Literature 10:
   Potentiation of the somatogenic and lactogenic activity of human growth hormone with monoclonal antibodies. (Aston R. et al., J. Endocrinol. 110: 381-388, 1986)
Non Patent Literature 11:
   Actions of monoclonal antibodies on the activity of human growth hormone (GH) in an in vitro bioassay. (Burbridge D. et al., Mol. Cell. Endocrinol. 174: 11-19, 2001)
Non Patent Literature 12:
   Characterization of monoclonal antibodies specific for the human growth hormone 22K and 20K isoforms. (Mellado M. et al., J. Clin. Endocrinol. Metab. 81:1613-1618, 1996)
Non Patent Literature 13:
   Monoclonal antibodies to human growth hormone modulate its biological properties. (Roguin L. P. et al., Mol. Immunol. 32: 399-405, 1995)
Non Patent Literature 14:
   Monoclonal antibodies against recombinant human growth hormone as probes to study immune function. (Lattuada D. et al., Hybridoma 15: 211-217, 1996)
Non Patent Literature 15:
   Characterization of monoclonal antibodies to human growth hormone (hGH) (Sato Y. et al., Tokyo Joshi Ika Daigaku Zasshi 54: 493-506, 1984)
Non Patent Literature 16:
   Novel Specific Bioassay for Serum Human Growth Hormone. (Ishikawa M. et al., J. Clin. Endocrinol. & Metab. 85: 4274-4279, 2000)
Non Patent Literature 17:
   Reagent from GeneTex, Inc.: Catalog Number: GTX52773, https://www.genetex.com/Product/Detail/Growth-Hormone-antibody-6J33/GTX52773

### Summary of Invention

In one embodiment, the present invention provides an antibody specifically binding to human GH or an antigen-binding fragment thereof.

The present invention encompasses the following embodiments.
(1) An antibody specifically binding to human growth hormone or an antigen-binding fragment thereof, comprising a heavy chain variable region (VH) and a light chain variable region (VL), wherein
   the VH comprises a VH complementarity-determining region (CDR) 1 (VHCDR1), a VHCDR2, and a VHCDR3,
   the VHCDR1 comprises an amino acid sequence set forth in SEQ ID NO: 5, or an amino acid sequence having substitution, addition, or deletion of one or two amino acids in the amino acid sequence set forth in SEQ ID NO: 5,
   the VHCDR2 comprises an amino acid sequence set forth in SEQ ID NO: 6, or an amino acid sequence having substitution, addition, or deletion of one or two amino acids in the amino acid sequence set forth in SEQ ID NO: 6,
   the VHCDR3 comprises an amino acid sequence set forth in SEQ ID NO: 7, or an amino acid sequence having substitution, addition, or deletion of one or two amino acids in the amino acid sequence set forth in SEQ ID NO: 7,
   the VL comprises a VL complementarity-determining region (CDR) 1 (VLCDR1), a VLCDR2, and a VLCDR3,
   the VLCDR1 comprises an amino acid sequence set forth in SEQ ID NO: 8, or an amino acid sequence having substitution, addition, or deletion of one or two amino acids in the amino acid sequence set forth in SEQ ID NO: 8,
   the VLCDR2 comprises an amino acid sequence set forth in SEQ ID NO: 9, or an amino acid sequence having substitution, addition, or deletion of one or two amino acids in the amino acid sequence set forth in SEQ ID NO: 9, and
   the VLCDR3 comprises an amino acid sequence set forth in SEQ ID NO: 10, or an amino acid sequence having substitution, addition, or deletion of one or two amino acids in the amino acid sequence set forth in SEQ ID NO: 10,
(2) An antibody specifically binding to human growth hormone or an antigen-binding fragment thereof, comprising a heavy chain variable region (VH) and a light chain variable region (VL), wherein
   the VH comprises an amino acid sequence set forth in SEQ ID NO: 5 as a VH complementarity-determining region (CDR) 1 (VHCDR1), an amino acid sequence set forth in SEQ ID NO: 6 as a VHCDR2, and an amino acid sequence set forth in SEQ ID NO: 7 as a VHCDR3, and
   the VL comprises an amino acid sequence set forth in SEQ ID NO: 8 as a VL complementarity-determining region (CDR) 1 (VLCDR1), an amino acid sequence set forth in SEQ ID NO: 9 as a VLCDR2, and an amino acid sequence set forth in SEQ ID NO: 10 as a VLCDR3.
(3) The antibody or antigen-binding fragment thereof according to (1) or (2), wherein the antibody or antigen-binding fragment thereof binds to human growth hormone at an equilibrium dissociation constant (K_{D}) of 1.0 × 10⁻⁸ mol/L or less as measured by a surface plasmon resonance method.
(4) The antibody or antigen-binding fragment thereof according to any of (1) to (3), wherein the antibody or antigen-binding fragment thereof neutralizes action of growth hormone.
(5) The antibody or antigen-binding fragment thereof according to any of (1) to (4), comprising: the VH comprising an amino acid sequence having substitution, addition, or deletion of one or several amino acids in regions other than the CDR1 to CDR3 in the amino acid sequence set forth in SEQ ID NO: 3, 11, 12, or 19, or an amino acid sequence having an identity of 90% or more with the amino acid sequence set forth in SEQ ID NO: 3, 11, 12, or 19, and/or
   the VL comprising an amino acid sequence having substitution, addition, or deletion of one or several amino acids in regions other than the CDR1 to CDR3 in an amino acid sequence set forth in SEQ ID NO: 4, 13, 14, or 20, or an amino acid sequence having an identity of 90% or more with the amino acid sequence set forth in SEQ ID NO: 4, 13, 14, or 20.
(6) The antibody or antigen-binding fragment thereof according to (5), comprising the VH having the amino acid sequence set forth in SEQ ID NO: 3, 11, 12, or 19, and/or the VL having the amino acid sequence set forth in SEQ ID NO: 4, 13, 14, or 20.
(7) The antibody or antigen-binding fragment thereof according to any of (1) to (6), wherein the antibody is a monoclonal antibody.
(8) The antibody or antigen-binding fragment thereof according to any of (1) to (7), wherein the antibody is a chimeric antibody, humanized antibody, veneered antibody, or fully human antibody.
(9) The antibody or antigen-binding fragment thereof according to any of (1) to (8), wherein the antibody comprises a heavy chain constant region comprising an amino acid sequence having substitution, addition, or deletion of one or several amino acids in an amino acid sequence set forth in SEQ ID NO: 17, or a heavy chain constant region comprising an amino acid sequence having an identity of 90% or more with the amino acid sequence set forth in SEQ ID NO: 17, and/or
   a light chain constant region comprising an amino acid sequence having substitution, addition, or deletion of one or several amino acids in an amino acid sequence set forth in SEQ ID NO: 18, or a light chain constant region comprising an amino acid sequence having an identity of 90% or more with the amino acid sequence set forth in SEQ ID NO: 18.
(10) The antibody or antigen-binding fragment thereof according to any of (1) to (9), wherein the antibody comprises the heavy chain constant region having the amino acid sequence set forth in SEQ ID NO: 17, and/or the light chain constant region having the amino acid sequence set forth in SEQ ID NO: 18.
(11) The antibody or antigen-binding fragment thereof according to any of (1) to (10), wherein the antigen-binding fragment is Fab, Fab', F(ab')2, Fv, or scFv.
(12) A nucleic acid encoding the antibody or antigen-binding fragment thereof according to any of (1) to (11).
(13) A vector comprising the nucleic acid according to (12) .
(14) A cell comprising the nucleic acid according to (12) or the vector according to (13).
(15) A medicine comprising the antibody or antigen-binding fragment thereof according to any of (1) to (11).
(16) The medicine according to (15), for decreasing the level of an insulin-like growth factor 1 (IGF-1) in the blood.
(17) The medicine according to (15) or (16), for treating and/or preventing a disease selected from the group consisting of acromegaly, gigantism, cancer, diabetic nephropathy, arthritis, and pneumonia.
(18) A method for decreasing the level of an insulin-like growth factor 1 (IGF-1) in the blood, comprising administration of a therapeutically effective amount of the antibody or antigen-binding fragment thereof according to any of (1) to (11).

In one embodiment, the present invention provides an antibody specifically binding to human GH or an antigen-binding fragment thereof.

### Brief Description of Drawings

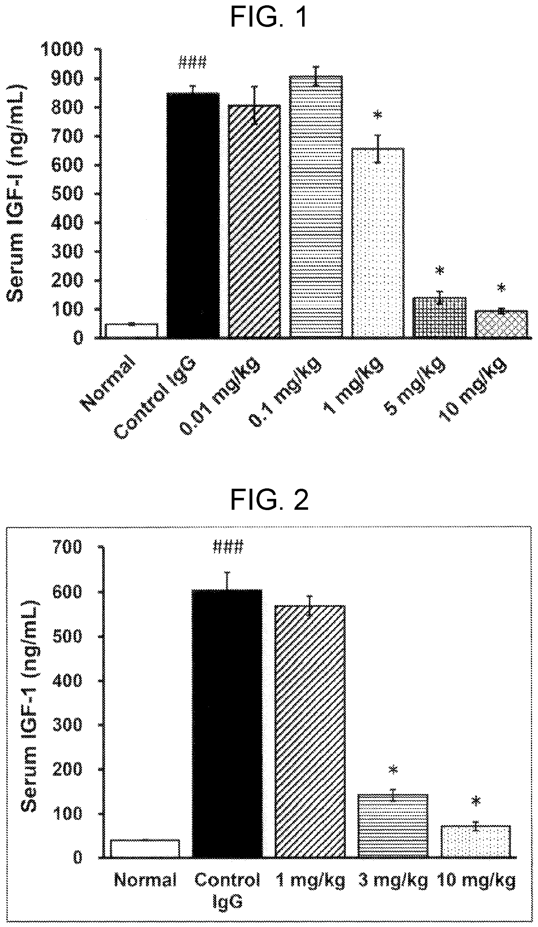

[Figure 1] Figure 1 shows serum IGF-1 values 2 days after administration of anti-GH mouse mAb (13H02) to GH supplemented-hyphophysectomized rats. The normal control group (Normal) is hypophysectomized rats without GH-supplementation; whereas the negative control group is GH-supplemented hypophysectomized rats to which control mouse IgG1 was administered.
[Figure 2] Figure 2 shows serum IGF-1 values 2 days after administration of anti-GH mouse human chimera mAb (CH-13H02) to the GH-supplemented hypophysectomized rats. The normal control group (Normal) is hypophysectomized rats without GH-supplementation; whereas the negative control group is GH-supplemented hypophysectomized rats to which control human IgG1 was administered.

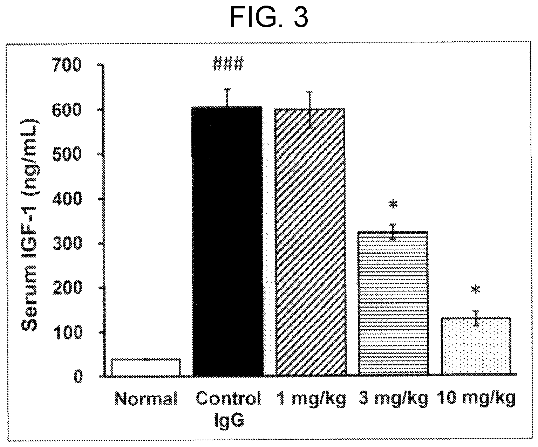

[Figure 3] Figure 3 shows serum IGF-1 values 2 days after administration of anti-GH humanized antibody Fc variant (Hu-13H02m) to GH-supplemented hypophysectomized rats. The normal control group (Normal) is hypophysectomized rats without GH-supplementation; whereas the negative control group is GH-supplemented hypophysectomized rats to which control human IgG1 was administered.

### Description of Embodiments

### 1. Antibody Which Binds to Growth Hormone

In one embodiment, the present invention provides an antibody specifically binding to human growth hormone (GH) or an antigen-binding fragment thereof (hereinafter, also referred to as anti-GH antibody).

"Growth hormone" is a hormone secreted from the cells in the anterior pituitary gland, and is involved in growth and metabolism of individuals. GH exhibits effects regarding growth such as elongation of bone and growth of muscle, and effects regarding metabolism such as metabolic promotion, increase in blood sugar level and maintenance of homeostasis, both by direct action on tissues and by stimulation of release of insulin-like growth factor-1 (IGF-1) through acting on a GH receptor (GHR) that is expressed in the liver and other tissues. IGF-1 is a peptide hormone having a similar structure to insulin, and is secreted in various tissues, including the liver. IGF-1 acts on numerous organs, and its physiological functions are diverse, including promotion of cell proliferation and differentiation, and protein synthesis and the like, and cell death-suppressing activity and the like.

The information of the amino acid sequence, mRNA sequence and the like of human GH is available from publicly accessible databases such as GenBank. In addition, the information of sequences of GH derived from other mammals such as mice or monkeys is also available from publicly accessible databases.

When simply referred to as "GH" in the present specification, it shall refer to a GH protein. In some cases, a gene encoding GH proteins may be simply referred to as GH. It will be apparent from the context for those skilled in the art when "GH" refers to a gene encoding GH proteins. Although GH as used herein is typically human GH, it may be GH derived from mammals other than humans (e.g., mice, rats, and monkeys).

As used herein, the term "antibody" refers to a glycoprotein comprising at least two heavy chains and two light chains bound to each other by disulfide bonds, and further comprising a J chain in the case of IgM. The heavy chain comprises a heavy chain variable region (VH) and a heavy chain constant region. There are γ chain, µ chain, α chain, δ chain, and ε chain in the heavy chain constant region, whose difference gives isotypes of IgG, IgM, IgA, IgD and IgE. The heavy chain constant region comprises three domains: CH1, CH2 and CH3; and further comprises CH4 in the case of IgE an IgM. The light chain comprises a light chain variable region (VL) and a light chain constant region, and the light chain constant region comprises one domain, CL. Two kinds referred to as λ chain and κ chain are present in the light chain constant region. The VH and VL regions are divided into four regions (FR1, FR2, FR3, and FR4) referred to as framework regions (FRs), which are relatively conserved among variable regions, and three hypervariable regions (CDR1, CDR2, and CDR3), which are referred to as complementarity-determining regions (CDRs) and contribute antigen-binding. VH comprises three CDRs and four FRs, which are arranged from the amino terminus to the carboxy terminus in the following order: FR1, CDR1 (CDRH1), FR2, CDR2 (CDRH2), FR3, CDR3 (CDRH3), and FR4. VL comprises three CDRs and four FRs, which are arranged from the amino terminus to the carboxy terminus in the following order: FR1, CDR1 (CDRL1), FR2, CDR2 (CDRL2), FR3, CDR3 (CDRL3), and FR4. CDRs can be determined according to the method of Kabat et al., for example (Kabat E. A. et al., 1992, Sequences of Proteins of Immunological Interest, the fifth edition, Public Health Service, NIH, Washington D.C.).

The "antibody" as used herein includes all classes and subclasses of intact immunoglobulins. The antibody described herein is, for example, IgG, more specifically, an antibody of which constant region may be human IgG1, a variant in which the effector function of IgG1 has been deleted, IgG2, IgG4 or IgG4 (S228P), and furthermore specifically, it may be IgG1 or a variant in which the IgG1 effector function of IgG1 has been deleted. In general, for the antibody that does not require any effector functions for exerting its functions, isotypes with decreased effector functions or variants in which the effector function has been deleted are preferable to be used in view of safety; however, the antibody described herein targets soluble GH, and therefore such isotypes or variants are less needed.

As used herein, examples of the "antigen-binding fragment" of the antibody include an isolated heavy chain, light chain, Fab, Fab', F(ab')2, Fv, scFv, and the like.

As used herein, the antibody or antigen-binding fragment thereof includes derivatives thereof. Examples of the derivative of the antibody or antigen-binding fragment thereof include an antibody in which an amino acid mutation has artificially been introduced into the constant region, an antibody in which the composition of the constant region domains has been modified, an antibody having two or more Fc per molecule, an antibody with glycan modification, a bispecific antibody, an antibody conjugate in which the antibody or antigen-binding fragment thereof is linked with other components, an abzyme, a tandem scFv, a bispecific tandem scFv, a trispecific tandem scFv, a diabody and the like.

The antibody described herein may be either polyclonal antibodies or a monoclonal antibody, and particularly, the monoclonal antibody is preferable.

The term "monoclonal antibody" (also described as "mAb") typically refers to an antibody obtained from a clone derived from a single antibody-producing cell. In other words, for mAb the amino acid sequences of individual antibodies constituting homogeneous or substantially homogeneous population are identical except possible mutations that may be present in a small amount and naturally occur. The mAb binds to a single antigenic determinant (epitope) on the antigen and has a high specificity to it. On the other hand, the polyclonal antibodies are a mixture consisting of multiple mAbs whose amino acid sequences are different in one or more sites.

As used herein, the mAb includes a fully human antibody and a non-human antibody.

The non-human antibody is an antibody other than the fully human antibody and includes, for example, an antibody derived from animals such as mouse, rat, rabbit or guinea pig, a chimeric antibody, a humanized antibody, and a veneered antibody.

The term "chimeric antibody" refers to an antibody in which its variable region sequences are derived from one species and its constant region sequences are derived from another species, for example, an antibody in which its variable region sequences are derived from a non-human animal antibody and its constant region sequences are derived from a human antibody, or the like.

The term "humanized antibody" refers to an antibody in which only its amino acid residues of the CDR sequences and a part of the frameworks in the variable regions are derived from a non-human animal antibody and the other sequences, that is, most part of the framework sequences and the entire constant region sequence are derived from a human antibody, or the like.

The term "veneered antibody" refers to one type of humanized antibodies in which part of and usually all of the CDRs of a non-human animal antibody and part of the variable region framework residues of the non-human animal are retained, but other variable region framework residues which can contribute as B- or T-cell epitopes, e.g., exposed residues (Padlan, Mol. Immunol. 28:489, 1991), are replaced with residues at positions corresponding to the human antibody sequence.

The term "fully human antibody" refers to an antibody entirely derived from a human antibody including CDR sequences.

The term "specifically binds" as used herein means that the antibody binds to a certain antigen with a significantly higher affinity than the affinity to a non-antigen substance (non-specific interaction). The affinity can be measured by a conventional method. Such a method is not particularly limited, and includes an enzyme-linked immunosorbent assay (ELISA), a surface plasmon resonance (SPR) method, and the like. When measuring the affinity in such a way, an antibody and/or an antigen can also be labeled with an enzyme, a fluorescent substance or the like, and the labeled substance can be measured to detect the affinity.

As parameters that represent the affinity, usually K_{D} (equilibrium dissociation constant), kₒₙ (binding rate constant), and k_{off} (dissociation rate constant) can be used.

As used herein, kₒₙ refers to the rate constant of the binding between the antibody and the antigen, and k_{off} refers to the rate constant of the dissociation of the antibody from the antigen-antibody complex. In addition, as used herein, K_{D} refers to the equilibrium dissociation constant of the antigen-antibody interaction, and it is calculated as k_{off}/kₒₙ.

In one embodiment, the antibody described herein binds to human GH with a K_{D} of, for example, 1 × 10⁻⁸ mol/L or less, 1 × 10⁻⁹ mol/L or less, 1 × 10⁻¹⁰ mol/L or less, 5 × 10⁻¹¹ mol/L or less, 2 × 10⁻¹¹ mol/L or less, and/or 1.0 × 10⁻¹³ or more, 1.0 × 10⁻¹² or more, 1.0 × 10⁻¹¹ or more as measured according to the method described in Example 12 in the present specification.

In one embodiment, the antibody described herein has a higher affinity to GH-22K among the isoforms of human GH (GH-22K and GH-20K).

As used herein, the term "cross-reactivity" refers to reactivity to another protein, for example, GH derived from a mammal other than human, a protein showing structural similarity to human GH (e.g., human placental lactogen (PL), and human prolactin (PRL)), and/or the other isoform of human GH (GH-20K) when the reactivity to human GH (GH-22K) used as an antigen in Examples in the present specification is taken as 100%.

The cross-reactivity of the anti-GH antibody can be measured in vitro, for example, by competitive ELISA described in Examples 3, 4 and 14 and by SPR described in Examples 13.

In one embodiment, the antibody or antigen-binding fragment thereof described herein comprises VH and VL; VH comprises a VH complementarity-determining region (CDR) 1 (VHCDR1), a VHCDR2, and a VHCDR3; and VL comprises VL complementarity-determining region (CDR) 1 (VLCDR1), VLCDR2, and VLCDR3. In one embodiment, the VHCDR1 comprises or consists of the amino acid sequence set forth in SEQ ID NO: 5, or an amino acid sequence having substitution, addition, or deletion of one or two amino acids in the amino acid sequence set forth in SEQ ID NO: 5. In one embodiment, the VHCDR2 comprises or consists of the amino acid sequence set forth in SEQ ID NO: 6, or an amino acid sequence having substitution, addition, or deletion of one or two amino acids in the amino acid sequence set forth in SEQ ID NO: 6. In one embodiment, the VHCDR3 comprises or consists of the amino acid sequence set forth in SEQ ID NO: 7, or an amino acid sequence having substitution, addition, or deletion of one or two amino acids in the amino acid sequence set forth in SEQ ID NO: 7. In one embodiment, the VLCDR1 comprises or consists of the amino acid sequence set forth in SEQ ID NO: 8, or an amino acid sequence having substitution, addition, or deletion of one or two amino acids in the amino acid sequence set forth in SEQ ID NO: 8. In one embodiment, the VLCDR2 comprises or consists of the amino acid sequence set forth in SEQ ID NO: 9, or an amino acid sequence having substitution, addition, or deletion of one or two amino acids in the amino acid sequence set forth in SEQ ID NO: 9. In one embodiment, the VLCDR3 comprises or consists of the amino acid sequence set forth in SEQ ID NO: 10, or an amino acid sequence having substitution, addition, or deletion of one or two amino acids in the amino acid sequence set forth in SEQ ID NO: 10. In one embodiment, the VHCDR1 comprises or consists of an amino acid sequence having substitution, addition, or deletion of one amino acid in the amino acid sequence set forth in SEQ ID NO: 5, the VHCDR2 comprises or consists of an amino acid sequence having substitution, addition, or deletion of one amino acid in the amino acid sequence set forth in SEQ ID NO: 6, the VHCDR3 comprises or consists of an amino acid sequence having substitution, addition, or deletion of one amino acid in the amino acid sequence set forth in SEQ ID NO: 7, the VLCDR1 comprises or consists of an amino acid sequence having substitution, addition, or deletion of one amino acid in the amino acid sequence set forth in SEQ ID NO: 8, the VLCDR2 comprises or consists of an amino acid sequence having substitution, addition, or deletion of one amino acid in the amino acid sequence set forth in SEQ ID NO: 9, and the VLCDR3 comprises or consists of an amino acid sequence having substitution, addition, or deletion of one amino acid in the amino acid sequence set forth in SEQ ID NO: 10.

In one embodiment, the antibody or antigen-binding fragment thereof described herein comprises the amino acid sequence in which the VH comprises an amino acid sequence set forth in SEQ ID NO: 5 as the VH complementarity-determining region (CDR) 1, the amino acid sequence set forth in SEQ ID NO: 6 as the VHCDR2, and the amino acid sequence set forth in SEQ ID NO: 7 as the VHCDR3, and the VL comprises the amino acid sequence set forth in SEQ ID NO: 8 as the VL complementarity-determining region (CDR) 1, the amino acid sequence set forth in SEQ ID NO: 9 as the VLCDR2, and the amino acid sequence set forth in SEQ ID NO: 10 as a VLCDR3. The VHCDR1 to CDR3 may consist of SEQ ID NOs: 5 to 7, respectively, and the VLCDR1 to CDR3 may consist of SEQ ID NOs: 8 to 10, respectively.

The sequences of the framework regions of the antibody or antigen-binding fragment thereof described herein are not limited as long as the antibody or the antigen-binding fragment thereof can specifically bind to GH. In one embodiment, the antibody or antigen-binding fragment thereof described herein comprises:
the VH comprising an amino acid sequence having substitution, addition, or deletion of one or several amino acids in regions other than the CDR1 to CDR3 in the amino acid sequence set forth in SEQ ID NO: 3, 11, 12, or 19, or an amino acid sequence having an identity of 90% or more, 91% or more, 92% or more, 93% or more, 94% or more, 95% or more, 96% or more, 97% or more, 98% or more, or 99% or more with the amino acid sequence set forth in SEQ ID NO: 3, 11, 12, or 19, and the regions of CDR1 to CDR3 consisting of SEQ ID NOs: 5 to 7, respectively; and/or
the VL comprising an amino acid sequence having substitution, addition, or deletion of one or several amino acids in regions other than the CDR1 to CDR3 in the amino acid sequence set forth in SEQ ID NO: 4, 13, 14, or 20, or an amino acid sequence having an identity of 90% or more, 91% or more, 92% or more, 93% or more, 94% or more, 95% or more, 96% or more, 97% or more, 98% or more, or 99% or more with the amino acid sequence set forth in SEQ ID NO: 4, 13, 14, or 20, and the regions of CDR1 to CDR3 consisting of SEQ ID NOs: 8 to 10, respectively.

In one embodiment, the antibody or antigen-binding fragment thereof described herein comprises:
the VH comprising an amino acid sequence having substitution, addition, or deletion of one or several amino acids in the amino acid sequence set forth in SEQ ID NO: 3, 11, 12, or 19, or an amino acid sequence having an identity of 90% or more, 91% or more, 92% or more, 93% or more, 94% or more, 95% or more, 96% or more, 97% or more, 98% or more, or 99% or more with the amino acid sequence set forth in SEQ ID NO: 3, 11, 12, or 19; and/or
the VL comprising an amino acid sequence having substitution, addition, or deletion of one or several amino acids in the amino acid sequence set forth in SEQ ID NO: 4, 13, 14, or 20, or an amino acid sequence having an identity of 90% or more, 91% or more, 92% or more, 93% or more, 94% or more, 95% or more, 96% or more, 97% or more, 98% or more, or 99% or more with the amino acid sequence set forth in SEQ ID NO: 4, 13, 14, or 20.

As used herein, "several" may be, for example, 2 to 10, 2 to 8, or 2 to 5, and for example, 2 or 3.

The term "identity" of the amino acid sequence herein means a proportion of matched amino acid residues. The identity of the amino acid sequence can be determined by the Basic Local Alignment Search Tool (BLAST) analysis method.

In one embodiment, the antibody or antigen-binding fragment thereof described herein comprises the VH having the amino acid sequence set forth in SEQ ID NO: 3, 11, 12, or 19, and/or the VL having the amino acid sequence set forth in SEQ ID NO: 4, 13, 14, or 20.

In one embodiment, the antibody or antigen-binding fragment thereof described herein comprises:
the heavy chain constant region comprising an amino acid sequence having substitution, addition, or deletion of one or several amino acids in the amino acid sequence set forth in SEQ ID NO: 17, or an amino acid sequence having an identity of 90% or more, 91% or more, 92% or more, 93% or more, 94% or more, 95% or more, 96% or more, 97% or more, 98% or more, or 99% or more with the amino acid sequence set forth in SEQ ID NO: 17; and/or
the light chain constant region comprising an amino acid sequence having substitution, addition, or deletion of one or several amino acids in the amino acid sequence set forth in SEQ ID NO: 18, or an amino acid sequence having an identity of 90% or more, 91% or more, 92% or more, 93% or more, 94% or more, 95% or more, 96% or more, 97% or more, 98% or more, or 99% or more with the amino acid sequence set forth in SEQ ID NO: 18. Here, a lysine residue at the C-terminus may be present or not in the amino acid sequence set forth in SEQ ID NO: 17.

In one embodiment, the antibody or antigen-binding fragment thereof described herein comprises the heavy chain constant region having the amino acid sequence set forth in SEQ ID NO: 17, and/or the light chain constant region having the amino acid sequence set forth in SEQ ID NO: 18. Here, a lysine residue at the C-terminus may be present or not in the amino acid sequence set forth in SEQ ID NO: 17.

The amino acid sequence in the constant region may comprise substitution that extends the blood half-life (see, e.g., Hinton P. R. et al., J. Biol. Chem. 279:6213-6216, 2004).

As modification of one or more amino acids in the heavy chain constant region for the purpose of extending the blood half-life of the antibody, for example, there are the methods referred to and described in US Patent Nos. 7,083,784, 7,217,797, 8,088,376. Examples described in the above-mentioned literatures include, in EU numbering in accordance with Kabat, one in which threonine at heavy chain position 250 is substituted with glutamine, one in which methionine at heavy chain position 428 is substituted with leucine, and one in which asparagine at heavy chain position 434 is substituted with serine, and multiple amino acid substitutions described above can be combined.

The antibody or antigen-binding fragment thereof comprising VH and/or VL comprising an amino acid sequence having substitution, addition, or deletion of one or several amino acids with respect to the original amino acid sequence, or an amino acid sequence having an identity of 90% or more with the original amino acid sequence, may have biological activity equivalent to that of the antibody having the original amino acid sequence or antigen-binding fragment thereof. Here, examples of the "equivalent biological activity" include (i) specific binding activity to GH, (ii) neutralizing activity to GH, (iii) IGF-1 production-suppressing activity when binding to GH, or (iv) any two or more or all of these.

In one embodiment, the antibody described herein neutralizes GH function. As used herein, the term "neutralization" refers to binding to a protein of interest to nullify or attenuate its function; and the term "neutralizing activity" refers to the degree of nullifying or attenuating the function. Since the antibody described herein specifically binds to GH, the neutralizing activity refers to the degree of nullification or attenuation of GH unless otherwise noted or apparent from the context that it is directed to other proteins. In one embodiment, the antibody described herein specifically binds to GH and blocks its interaction with GHR to decrease the IGF-1 level in plasma and/or tissue.

The neutralizing activity of the antibody described herein can be measured, for example, in vitro (e.g., with GH-GHR binding inhibition assay or GH-dependent Nb₂ cell proliferation inhibition assay described in Example 2 in the specification of the present application). In one embodiment, the antibody described herein has an IC₅₀ of 10,000 pmol/L or less, 1,000 pmol/L or less, or 100 pmol/L or less, and/or 1 pmol/L or more, 0.1 pmol/L or more, or 0.01 pmol/L or more in the GH-GHR binding inhibition assay described in Example 2. In one embodiment, the antibody described herein has an IC₅₀ of 1,000 pmol/L or less, 100 pmol/L or less, or 10 pmol/L or less, and/or 1 pmol/L or more, 0.1 pmol/L or more, or 0.01 pmol/L or more in the GH-dependent Nb₂ cell proliferation inhibition assay described in Example 2.

The antibody described herein may be used to reduce the serum IGF-1 concentration, to alleviate and treat various symptoms caused by GH hypersecretion, or may be prophylactically used to a human who will exhibit some symptoms in the future caused by GH excess. The antibody described herein may be used to a patient who does not respond to conventional therapies against GH hypersecretion. In one embodiment, the antibody described herein can be used to treat and/or prevent a disorder associated with GH hypersecretion, including, for example, acromegaly, gigantism, a certain cancer, diabetic nephropathy, arthritis, and pneumonia.

In one embodiment, the antibody described herein may have excellent (e.g., equal or greater than pegvisomant) drug efficacy, safety and/or good blood kinetics.

In one embodiment, the antibody described herein decreases the serum IGF-1 values in vivo. For example, the antibody described herein dose-dependently decreases the increased serum IGF-1 values by continuous administration of exogenous human GH to rats which lack endogenous GH due to hypophysectomy. In one embodiment, the antibody described herein also neutralizes the endogenous GH and continuously decrease the serum IGF-1 values by being administered to mammals such as monkeys.

In one embodiment, the antibody described herein has little or no toxicity to mammals.

In one embodiment, the antibody described herein exhibits good physical properties (e.g., at least one of water solubility, acid stability, thermal stability, and hydrophobic interaction).

### 2. Preparation of the Antibody Described Herein

The antibody described herein can be selectively acquired from B-cells obtained by immunizing animals with antigen, by various methods known in the art, including hybridoma technologies, a variety of display technologies, or single-cell screening technologies. In addition, a fully human monoclonal antibody can be produced by using genetically modified animals in which human antibody genes have been incorporated for immunization of antigen or using antibody display libraries constructed from human antibody gene pools.

The hybridoma technology is a technique in which antibody-producing B-cells obtained by immunizing animals such as mice or rats with antigens are fused with myeloma cells to make them immortalized to obtain monoclonal hybridoma cell lines, from which an antibody-producing cell line of interest is selectively acquired to obtain a monoclonal antibody. The techniques are not limited, and can be performed, for example, as described below.

As the antigens for producing the antibody described herein, human GH or a fragment thereof can be used. Animals such as mice or rats are immunized multiple times with these antigens in combination with a variety of adjuvants, and the animals whose serum antibody titer has been increased are finally immunized with the antigen, and then their spleens or lymph nodes are resected to collect lymphocytes including antibody-producing B-cells. The lymphocytes (B-cells) are fused with myeloma cell lines by polyethylene glycol or with an electric cell fusion apparatus, and the cells are cultured in a selective medium in which only the fused cells of both can survive (e.g., selective medium comprising hypoxanthine-aminopterin-thymidine (HAT)) to establish hybridoma. The monoclonal hybridoma thus obtained is appropriately cultured, and the human GH binding activity and human GH neutralizing activity of the resultant culture supernatant are measured, whereby hybridoma producing human GH-specific neutralizing antibody can be selectively acquired. The details of the hybridoma prodcution techniques are known and for example, can be found in Koehler, G. et al., Nature 256: 495-497, 1975, Hnasko R. M. et al., Methods Mol. Biol. 1318: 15-28, 2015.

Examples of the antibody production method by the display technology include phage display, yeast display, cDNA display, ribosome display, and animal cell display. Specifically, antibody fragments (e.g., scFv or Fab) encoded by the genes isolated from the B-cell pool that is isolated from the immunized animals described in the above hybridoma technology, non-immunized animals, disease patients or healthy humans, are presented on a variety of display systems to construct a library. From this library, antibodies having affinity to antigens and genes thereof are concentrated, amplified and/or isolated by a selection method called biopanning, and thereby an antibody of interest can be obtained. The details of the display techniques are known and for example, can be found in US Patent Nos. 5,223,409, 5,885,793, 6,582,915, 5,969,108 and 6,172,197, 5,821,047, 6,706,484, 6,753,136, and Winter G. P. et al., Annu. Rev. Immunol. 12:433-455, 1994, Rothe C. et al., J. Mol. Biol. 376: 1182-1200, 2008, Scholler N., Methods Mol. Biol. 1827: 211-233, 2018, Yamaguchi J. et al., Nucleic Acids Res. 37: e108, 2009, Dreier B. et al., Methods Mol. Biol. 1827: 235-268, 2018, Zhou C. et al., Methods Mol. Biol. 907: 293-302, 2012.

Examples of the antibody production method by the single B-cell screening technology include a method in which the B-cells isolated from the immunized animals described above or disease patients are, for example, seeded into a tiny cell at one cell/cell, and the antibody-producing B-cell of interest is selected by detecting the activity of the antibody secreted from each cell by the fluorescence response, or a method in which the B-cells are reacted with a fluorescent labeled antigen, and fluorescently stained B cells producing antibody of interest are sorted to 1 cell/well by the fluorescent-activated cell sorting (FACS), and thereby an antibody of interest can be obtained by isolating antibody genes from the B-cells thus collected. The details of the single B-cell screening technology are known and for example, can be found in Love J. C. et al., Nat. Biotechnol. 24:703-707, 2006, Tiller T. et al., J. Immunol. Methods 29:112-124, 2008, Jin A. et al., Nat. Med. 15:1088-1092, 2009, Starkie D. O. et al., PLoS ONE 11: 20152282, 2016, Winters A. et al., mAbs 11:1025-1035, 2019, Josephides D. et al., SLAS Technol. 25:177-189, 2020.

A Chimeric antibody can be produced, for example, by linking non-human antibody variable region sequence with human antibody constant region sequence by genetic engineering technique, and preparing the resultant with methods described later. The details of the chimeric antibody production technology are known and for example, can be found in US Patent No. 5,482,856, and Morrison S. L. et al., Proc. Natl. Acad. Sci. USA 81: 6851-6855, 1984.

A humanized antibody can be produced, for example, by grafting part of and usually all of the CDRs of a non-human antibody, and a part of the non-human variable region framework residues to the human antibody variable region having high homology to the non-human antibody variable region sequence. The details of the humanized antibody production technology are known and for example, can be found in US Patent Nos. 5,530,101, 5,585,089, 5,225,539, 6,407,213, 5,859,205, 6,881,557, and Queen C. L. et al., Proc. Natl. Acad. Sci. USA 86: 10029-10033, 1989, Tsurushita N. et al., Methods 36: 69-83, 2005, Choi Y. et al., MAbs 7: 1045-1057, 2015.

A veneered antibody can be prepared, for example, by retaining part of and usually all of the CDRs of the non-human antibody and a part of the non-human variable region framework residues, and substituting the other variable region framework resides which can contribute as B- or T-cell epitopes (e.g., exposed residues) (Padlan, Mol. Immunol. 28:489, 1991) with residues from positions corresponding to the human antibody sequence, respectively. The details of the veneered antibody preparation technology are known and for example, can be found in US Patent No. 5,585,089, and Riechmann L. et 1., Nature 322: 323-327, 1988.

A fully human antibody can be produced, for example, by using the aforementioned monoclonal antibody production technology from the B-cells obtained by immunizing transgenic animals carrying human antibody gene with antigen. In addition, the fully human antibody can be produced from a variety of antibody display libraries prepared from the B-cell pool isolated from disease patients or healthy humans. The details of the fully human antibody production technology are known and for example, can be found in Green L. L. et al., Nat. Genet. 7: 13-21, 1994, Lonberg N. et al., Nature 368: 856-859, 1994, Mendez M. J. et al., Nat. Genet. 15: 146-156, 1997, Ishida I. et al., Cloning Stem Cells 4: 91-102, 2002, Osborn M. J. et al., J. Immunol. 190: 1481-1490, 2013, Lee E. C. et al, Nat. Biotechnol. 32: 356-363, 2014, Murphy A. J. et al., Proc. Natl. Acad. Sci. USA 111: 5153-5158, 2014, Winter G. P. et al., Annu. Rev. Immunol. 12:433-455, 1994, Rothe C. et al., J. Mol. Biol. 376: 1182-1200, 2008, and US Patent Nos. 5,223,409, 5,885,793, 6,582,915, 5,969,108, and 6,172,197, 5,821,047, 6,706,484, 6,753,136.

The heavy chain and light chain variable regions of the chimeric antibody, humanized antibody, veneered antibody, or fully human antibody can be linked to, for example, any constant region of the human heavy chain and human light chain, respectively. The selection of isotype of the heavy chain constant region depends on whether complement-dependent cytotoxicity and antibody-dependent cellular cytotoxicity is desired as the target or the mechanism of action of antibody. For example, generally, human IgG1 and IgG3 have strong complement-dependent cytotoxicity and antibody-dependent cellular cytotoxicity, and human IgG2 and IgG4 have weak effector functions thereof. In addition, as the light chain constant region, either of lambda or kappa can be selected. The human constant region shows allotype variation among different individuals but includes any substitution of the constant region having any natural allotype or of residues occupying polymorphic position of natural allotype. One or more amino acids at the amino or carboxy terminus of the light chain and/or heavy chain of the antibody (e.g., lysine at the C-terminus of the heavy chain) may be deleted or derivatized in a certain proportion of molecules or all molecules.

The blood half-life of the antibody described herein can be extended by substituting the amino acid residues in the heavy chain constant region. Examples of the typical substitution for extending the blood half-life include for example, with EU numbering in accordance with Kabat, a method in which threonine at heavy chain position 250 is substituted with glutamine, a method in which methionine at heavy chain position 428 is substituted with leucine, and a method in which asparagine at heavy chain position 434 is substituted with serine, and the amino acid substitution described above can be combined with two or more thereof (see, US Patent Nos. 7,083,784, 7,217,797, and 8,088,376).

### 3. Production of the Antibody Described Herein

In one embodiment, the present invention provides a method for producing the antibody or antigen-binding fragment described herein.

In one embodiment, the present invention provides an isolated nucleic acid encoding the antibody or antigen-binding fragment described herein. The nucleic acid molecule may be RNA or DNA. The nucleic acid described herein can be used to produce the antibody or antigen-binding fragment described herein. The nucleic acid encoding the antibody or antigen-binding fragment described herein can be isolated from, for example, the B-cells, hybridomas, isolated clones of various antibody display (e.g., phages, yeasts, animal cells, cDNAs) that produce the antibody described herein, and the sequence can be determined. For example, the genes encoding the amino acid sequence of the heavy chain and or light chain variable regions of the antibody can be isolated and the sequence can be determined by using oligonucleotide probes that specifically bind to the constant region sequence thereof.

In addition, the antibody with known amino acid sequence (including chimeric antibody, humanized antibody, and veneered antibody, and fully-human antibody, as well as an antibody having modification of amino acids in the variable region or constant region) can be produced by using nucleic acids encoding the amino acid sequence thereof with a known gene recombinant technologies, or by chemical synthesis. The antibody or antigen-binding fragment produced with a gene recombinant technologies is also referred to as recombinant antibody or recombinant antigen-binding fragment.

In one embodiment, the present invention provides a vector comprising nucleic acid encoding the antibody or antigen-binding fragment described herein. The vector comprising nucleic acid encoding the antibody or antigen-binding fragment described herein, for example, typically includes an expression regulation sequence (e.g., replication initiation point, promoter, enhancer (Queen C. et al., Immunol. Rev. 89:49-68, 1986), and necessary processing information site (e.g., including ribosome binding site, RNA splicing site, polyadenylation site and transcription terminator sequence)) that is feasibly linked to the nucleic acid that encodes the antibody or antigen-binding fragment and that can be transformed or transfected into a eukaryotic host cell, and the vector can be easily constructed by a known gene recombinant technologies.

In one embodiment, the present invention provides a nucleic acid encoding the antibody or antigen-binding fragment described herein, or a cell comprising a vector comprising the nucleic acid. For example, methods for producing a hybridoma cell comprising a nucleic acid that encodes the antibody or antigen-binding fragment described herein are as described above.

For example, cells that express recombinant antibodies or recombinant antigen-binding fragments (hereinafter, also referred to as "recombinant cells") can be obtained by transfecting a vector comprising the nucleic acid encoding the antibody or antigen-binding fragment into a host cell to allow them to be expressed transiently or stably. Examples of the host cell include an Escherichia coli cell, yeast cell, insect cell, and mammalian cell, and the preferred host cell among them include a mammalian cell, such as a human HEK293 fetal kidney-derived cell, monkey COS cell, Chinese hamster ovary (CHO) cell, and mouse myeloma cell (Sp2/0 and NS0). The details of the technology for producing cells that express the recombinant antibody or recombinant antigen-binding fragment are known.

The recombinant antibody or recombinant antigen-binding fragment can be produced by culturing the above-mentioned recombinant cells and purifying them from a cell extract or culture supernatant according to a standard protein purification technique including a variety of column chromatography and ultraconcentration. The details of the technology for purifying the recombinant antibody or recombinant antigen-binding fragment are known.

The above-mentioned method for producing an antibody with a known amino acid sequence, method for constructing a vector comprising a nucleic acid that encodes an antibody or antigen-binding fragment, method for producing a recombinant cell, and method for purifying a recombinant antibody or recombinant antigen-binding fragment can be found in, for example, Ossipow V. et al., Monoclonal Antibodies, Methods Mol. Biol. 1131 (the second edition), 2014.

Further, the recombinant antibody or recombinant antigen-binding fragment can be produced by expressing them in a genetically modified plant (e.g., tobacco or duckweed) or in milk of a genetically modified animal (e.g., cow or goat), in which a vector comprising the nucleic acid that encodes these proteins has been integrated into the chromosome, and by purifying according to a method similar to the one mentioned above. For methods for producing an antibody-genetically modified plant and antibody-genetically modified animal, see, for example, Perdigones A. S. et al., Cell Engineering 7: 143-164, 2011, Pollock D. P. et al., J. Immunol. Methods 231: 147-157, 1999.

The antigen-binding fragment can be produced by enzymatic digestion of antibody or (for example, in case of a short polypeptide with 50 amino acids or less) chemical synthesis, in addition to the above method for producing an antibody. The methods of the enzymatic digestion of antibody and chemical synthesis are known. For the enzymatic digestion, the details of techniques of preparing Fab by partial digestion of IgG with papain or the like is known, and can be found in, for example, Zao Y. et al., Protein Expression and Purification 67: 182-189, 2009. For the chemical synthesis, the production can be made with, for example, an automatic polypeptide synthesizer using a solid-phase method, and the method can be found in, for example, US Patent Nos. 5,807,715, 4,816,567, and 6,331,415.

### 4. Medicines and Methods

In one embodiment, the present invention relates to a medicine comprising the antibody or antigen-binding fragment described herein.

In one embodiment, the antibody or antigen-binding fragment, or the medicine described herein can be used to decrease the level of IGF-1 in blood. In one embodiment, the antibody or antigen-binding fragment, or the medicine described herein can be used to prevent and/or treat symptoms related to excess GH and/or excess IGF-1 in individuals.

Examples of the symptoms related to excess GH and/or excess IGF-1 include acromegaly, gigantism, pituitary gigantism, glucose intolerance, diabetic nephropathy, sleep apnea syndrome, decreased energy, irregular menstruation, cancer, increased tumor incidence, malocclusion, excessive sweating, numbness of limbs, arthritis, joint pain due to osteoarthritis, and pneumonia. The symptoms related to excess GH and/or excess IGF-1 are preferably acromegaly, gigantism, glucose intolerance, diabetic nephropathy, and sleep apnea syndrome, and more preferably acromegaly and gigantism.

In one embodiment, the antibody or antigen-binding fragment, or the medicine described herein decreases the blood IGF-1 level. The blood IGF-1 level may be, for example, 90% or less, 80% or less, 70% or less, 60% or less, 50% or less, 50% or less, 40% or less, 30% or less, 20% or less, or 10% or less of before administration.

In one embodiment, therapeutical administration of the antibody or antigen-binding fragment, or the medicine described herein mitigates or ameliorates one or two or more symptoms of acromegaly, and/or reduces the onset. The symptoms of acromegaly are not particularly limited, and include excess IGF-1, excessive sweating, offensive body odor, thicker skin, darkening of skin, oily skin, small wart (skin tag), fatigue, muscle weakness, lowering voice pitch due to swollen vocal cord and expansion of paranasal sinus and/or proliferation of cartilage in pharynx, heavy snoring, sleep apnea, visual disorder, headache, enlarged tongue, back pain, joint pain, reduced joint range of motion, menstrual cycle irregularity, decreased sexuality, erectile dysfunction, hand hypertrophy, foot hypertrophy, largely expanded face, lower dentition protruding over the upper dentition due to mandibular protrusion (reversed occlusion), liver hypertrophy, heart hypertrophy, kidney hypertrophy, spleen hypertrophy, enlarged chest (barrel chest), increased coarse body hair, inappropriate processing of sugar in food, diabetes, hypertension, increased urinary calcium, nephrolithiasis, gallstone, thyroid edema (goiter), heart disease, arthritis, precancerous growth in colon (polyp), carpal tunnel syndrome, hypopituitarism, uterine fibroid, peripheral neuropathy that results in intraneural fibroplasia, necrosis and/or erosion of proliferated articular cartilage, hyperphosphatemia and spinal cord compression.

The clinical criteria and prognosis indicator for acromegaly are well known in the art, and diagnosis or evaluation of acromegaly has been established. Individuals suitable for treatment and/or prevention by the antibody or antigen-binding fragment, or the medicine described herein may be selected according to the abovementioned clinical criteria or evaluation. The evaluation of severity of acromegaly may be conducted based on known tests such as measurement of blood IGF-1 level, measurement of GH before and after oral glucose test, and magnetic resonance imaging (MRI) on the head for detecting pituitary tumor. The mitigation, amelioration, or regulation of symptoms of acromegaly, reduction of its onset, or delay of its development or progression may be measured by testing the blood IGF-1 level.

In one embodiment, therapeutical administration of the antibody or antigen-binding fragment, or the medicine described herein mitigates or ameliorates one or two or more symptoms of gigantism, and/or reduce the onset. The symptoms of gigantism are not particularly limited, and include excess IGF-1, excess body height growth, excess muscle growth, excess organ growth, delay of puberty, double vision, difficulty in peripheral vision, forehead protrusion, protruded chin, headache, increase in sweating, irregular menstruation, big hand, big foot, thick finger, thick toe, release of breastmilk, thicker face, debility, adrenal insufficiency, diabetes insipidus, hypogonadism, and hypothyroidism.

The clinical criteria and prognosis indication for gigantism are well known in the art, and diagnosis or evaluation of gigantism has been established. Individuals suitable for treatment and/or prevention by the antibody or antigen-binding fragment, or the medicine described herein may be selected according to the clinical criteria or evaluation. The evaluation of severity of gigantism may be conducted based on known tests such as computer tomography (CT) or MRI on the head for detecting pituitary tumor, measurement of GH before and after oral glucose test, measurement of blood prolactin, measurement of blood IGF-1, measurement of blood cortisol, measurement of blood estradiol, measurement of blood testosterone, and measurement of thyroid hormone. The mitigation, amelioration or regulation of symptoms of gigantism, reduction of its onset, or delay of its development or progression may be measured by testing the blood IGF-1 level.

The term "treatment" as used herein includes improvement of symptoms related to excess GH, as well as all the medical treatment related to excess GH, such as improved symptoms being kept, recurrence suppression, reduction in other treatment, and also includes improvement in quality of life of people suffering from symptoms due to excess GH. The term "prevention" as used herein includes preventing the occurrence of symptoms related to excess GH or reducing the risk thereof.

In one embodiment, the medicine described herein further comprises a pharmaceutically acceptable carrier (e.g., excipient, expander, binder, and lubricant), and/or a known additive (e.g., buffering agent, isotonic agent, chelating agent, colorant, preservative, fragrance, flavoring agent, and sweetening agent).

A pharmaceutical composition described herein can comprise one or two or more other agents.

The medicine described herein is generally systemically or locally administered in an oral or parenteral form. The medicine described herein can be administered to a living organism by intravenous, intramuscular, intraperitoneal, or subcutaneous injection, a suppository, a topical agent, or the like. The amount of the medicine described herein to be administered differs depending on the age, body weight, symptoms, therapeutical effects, administration method, processing time or the like, and varies under various conditions. For example, it can be administered to an individual in an amount of 0.0001 to 100 mg/kg, and preferably 0.01 to 100 mg/kg per day as a medicine or as an active ingredient.

The medicine described herein can be made into pharmaceutically acceptable formulation. The formulation can be made into an injection of an aseptic solution, suspending solution, freeze-drying formulation, or tablets, granules, powders, capsules, emulsion, suspending agent, syrup, or the like, according to means to be generally performed.

The medicine described herein can be used alone or in combination with other conventional treatment methods. In this case, administration timing and treatment timing of the medicine described herein and other agents are not limited, and these may be administered to a target subject simultaneously or with a time lag.

In one embodiment, the present invention relates to a method for decreasing the level of an IGF-1 in the blood, comprising administering a therapeutically effective amount of the antibody or antigen-binding fragment thereof. The method may be for treating and/or preventing symptoms related to the excess GH and/or excess IGF-1 mentioned above. The method may further comprise identifying an individual at risk from the diseases related to excess GH and/or excess IGF-1, evaluating an individual for risk factors, and/or diagnosing an individual for their symptoms.

### Examples

Hereinafter, the present embodiment is described in more detail with Examples; however, the present invention is not limited to these, and it may be modified in a range without departing from the scope of the present invention.

### <Example 1: Measurement Method of GH-Binding Activity >

Recombinant human GH (Pharmaceutical and Medical Device Regulatory Science Society of Japan) diluted with Dulbecco's phosphate buffer solution (PBS) to 5 µg/mL was immobilized at 50 µL/well on a 96-well half-area immunoplate (Corning Incorporated), and the plate was blocked with PBS comprising 1% bovine serum albumin (BSA). Next, a positive control (anti-GH mouse mAb (MAB1067, R&D Systems, Inc.)) diluted with assay buffer (PBS comprising 0.2% BSA and 0.05% Tween 20) or an anti-GH mouse mAb-comprising sample was added at 50 µL/well, and the plate was left still at room temperature for 2 hours. The plate was washed with washing buffer (PBS comprising 0.05% Tween 20), followed by adding, at 50 µL/well, horseradish peroxidase (HRP)-labeled goat anti-mouse IgG antibody (Jackson ImmunoResearch Laboratories Inc.) which was diluted with assay buffer to 0.1 µg/mL, and the plate was left still at room temperature for 2 hours. The plate was further washed with washing buffer, followed by adding 3,3',5,5'-tetramethyl benzidine (TMB) solution as a substrate of HRP at 50 µL/well, and allowed to react at room temperature for 10 minutes. The reaction was stopped by adding an aqueous solution of 0.5 mol/L H₂SO₄, and then the absorbance at 450 nm was measured with a plate reader.

### <Example 2: Measurement Method of GH-Neutralizing Activity>

For the purpose of evaluating the GH neutralizing activity of the anti-GH antibody, (a) GH-GH receptor (GHR) binding inhibition assay, and (b) GH-dependent Nb₂ cell proliferation inhibition assay were established.

### (a) GH-GHR Binding Inhibition Assay

The GH-GHR binding inhibitory activity was measured by calculating the inhibition rate of binding signal of biotin-labeled GH to GHR extracellular region protein-human Fc fusion (GHR-hFc) which was immobilized on an ELISA plate via anti-human Fcy mouse antibody. The biotin-labeled GH was prepared by labeling recombinant GH with EZ-Link Micro NHS-PEG4-Biotinylation kit (Thermo Fisher Scientific Inc.). The specific method is as follows. Anti-human Fcy mouse antibody (Jackson ImmunoResearch Laboratories, Inc.) diluted to 1 µg/mL PBS was immobilized at 50 µL/well on a 96-well half-area immunoplate, and the plate was blocked with PBS comprising 1% BSA. Next, GHR-hFc (R&D Systems, Inc.) diluted with assay buffer (PBS comprising 0.2% BSA and 0.05% Tween 20) to 0.1 µg/mL was added at 50 µE/well to be captured on the plate. The plate was washed with washing buffer (PBS comprising 0.05% Tween 20), followed by mixing the equivalent amount of biotin-labeled GH diluted with assay buffer to final concentration of 5.5 ng/mL and a sample (anti-GH mouse mAb-comprising sample) diluted with assay buffer, and adding the resultant mixture at 50 µL/well, and the plate was left still at room temperature for 2 hours. As a positive control, non-labeled GH or anti-GH mouse mAb (MAB1067) was used instead of the sample. The plate was washed with washing buffer 4 times, followed by adding, at 50 µL/well, HRP-labeled streptavidin (Jackson ImmunoResearch Laboratories Inc.) diluted with assay buffer to 0.1 µg/mL, and the plate was left still at room temperature for 2 hours. The plate was further washed 6 times, followed by adding TMB solution as a substrate of HRP at 50 µL/well, and allowed to react at room temperature for 10 minutes. The reaction was stopped by adding an aqueous solution of 0.5 mol/L H₂SO₄, and then the absorbance at 450 nm was measured with a plate reader. IC₅₀ was calculated by converting GH-GHR binding inhibition rate of each sample, as the absorbance when adding the biotin-labeled GH only to be 0% inhibition, and the absorbance when adding no biotin-labeled GH to be 100% inhibition. IC₅₀ of the positive control antibody MAB1067 was 6,860 pmol/L under the above conditions.

### (b) GH-Dependent Nb₂ Cell Proliferation Inhibition Assay

As a method for measuring cell-based GH-neutralizing activity, the Nb₂ rat lymphoma cell line proliferation assay, which is known as a standard GH activity-measuring method was used (Omae Y. et al., Endocrinol Japon 36, 9-13, 1989). The specific method is as follows. Nb2-11 cell line (EC90741101, ECACC) conditioned to a Fischer's Medium comprising 10% fetal bovine serum and 10% horse serum (HS) was cultured in a Fisher's Medium comprising 1% HS for 24 hours with a CO₂ incubator (37°C, 5% CO₂) to stand in serum starvation condition. The cells were seeded on 96-well culture plate at 2 × 10⁴ cell/well, followed by adding each of the human GH (Pharmaceutical and Medical Device Regulatory Science Society of Japan) diluted with PBS comprising 0.1% BSA to the final concentration of 9.1 pmol/L (EC₈₀: 80% effective concentration) and an anti-GH mAb sample (5 nmol/L) diluted with the same buffer to 5⁰ to ⁷-fold at 10 µL/well. As a positive control, anti-GH mouse mAb (MAB1067) was used instead of the sample. The cells were cultured for 72 hours in the CO₂ incubator, followed by adding WST-8 (Cell counting kit-8; DOJINDO LABORATORIES) at 10 µL/well to allow to react for about 3 hours at 37°C, and then the absorbance at 450 nm was measured with a plate reader as an index of viable cell counts. IC₅₀ was calculated, as the absorbance when adding no GH and no anti-GH antibody to be 100% inhibition, and the absorbance when adding GH and no anti-GH antibody to be 0% inhibition. IC₅₀ of the positive control antibody MAB1067 was 21,500 pmol/L under the above conditions.

### <Example 3: Cross-Reactivity of Anti-GH Mouse mAb to Experimental Animal-Derived GH>

The cross-reactivities of anti-GH mouse mAb to GH derived from monkey, mouse and rat were measured with a competitive ELISA. The specific method is as follows.

### (a) Preparation of Recombinant Cynomolgus Monkey GH

The DNA sequence (NM_001290284.1) of monkey GH cloned from cynomolgus monkey pituitary gland cDNA was inserted into a pIEx-4 insect cell expression vector (Merck Millipore). The vector was transfected into a HighFive cell line (Invitrogen), and the cells were cultured with rotation for 72 hours at 28°C, and then the culture supernatant was collected by centrifugation. The culture supernatant was subjected to CaptureSelect hGH Affinity Matrix (Thermo Fisher Scientific Inc.) equilibrated with 20 mmol/L Tris-hydrochloride buffer solution comprising 150 mmol/L NaCl (pH 8.0), and the resin was washed with the same buffer solution comprising 500 mmol/L NaCl, and then monkey GH adsorbed on a carrier was eluted with 20 mmol/L citrate buffer solution comprising 150 mmol/L NaCl (pH 3.0). The eluate was dialyzed against 20 mmol/L Tris-hydrochloride buffer solution comprising 150 mmol/L NaCl and 10% glycerol (pH 8.0) to obtain a final sample (monkey GH). The purity of the monkey GH assessed by SDS-PAGE and size-exclusion chromatography using Superdex 75 Increase 10/300 GL (GE Healthcare Inc.) was > 85%, and it showed the activity of EC₅₀ = 9.8 pmol/L in the Nb₂ cell line proliferation assay described in Example 2(b).

### (b) Cross-Reactivity Test

Recombinant human GH (Pharmaceutical and Medical Device Regulatory Science Society of Japan) diluted with PBS to 5 µg/mL was immobilized at 50 µL/well on a 96-well half-area immunoplate (Corning Incorporated), and the plate was blocked with PBS comprising 1% BSA. Next, human GH, cynomolgus monkey GH prepared as above, mouse GH (LifeSpan BioSciences, Inc.), or rat GH (Protein Laboratories Rehovot Ltd.) serially diluted with assay buffer (PBS comprising 0.2% BSA and 0.05% Tween 20) was mixed together with anti-GH mouse mAb diluted with the same buffer to the final concentration of 30 ng/mL, and the mixture was added at 50 µL/well, and the plate was left still at room temperature for 2 hours. The plate was washed with washing buffer (PBS comprising comprising 0.05% Tween 20), followed by adding, at 50 µL/well, HRP-labeled goat anti-mouse IgG antibody (Jackson ImmunoResearch Laboratories Inc.) diluted with assay buffer to 0.1 µg/mL, and the plate was left still at room temperature for 2 hours. The plate was further washed with washing buffer, followed by adding TMB solution as a substrate of HRP at 50 µL/well, and allowed to react with the enzyme at room temperature. Ten minutes later, the reaction was stopped by adding 0.5 mol/L H₂SO₄, and then the absorbance at 450 nm was measured with a plate reader. IC₂₀ (nmol/L) values of the anti-GH mouse mAb were determined by concentration-dependent curves of human GH and experimental animal-derived GHs to calculate the cross-reactivity to each experimental animal-derived GH by the following formula 1. Cross-reactivity to experimental animal-derived GH (%) = IC20 (human GH) / IC20 (experimental animal-derived GH) x 100

### <Example 4: Cross-Reactivity Test of Anti-GH Mouse mAb to GH-Analogous Protein (protein having structural similarity to GH)>

The cross-reactivities of the anti-GH mouse mAb to proteins having structural similarity to GH (placental lactogen (PL), prolactin (PRL)) were measured with a competitive ELISA. The specific method is as follows.

Recombinant human GH (Pharmaceutical and Medical Device Regulatory Science Society of Japan) diluted with PBS to 5 µg/mL was immobilized at 50 µL/well on a 96-well half-area immunoplate (Corning Incorporated), and the plate was blocked with PBS comprising 1% BSA. Next, human GH, PL (Protein Laboratories Rehovot Ltd.), or PRL (BBT Boster Immunoleader) serially diluted with assay buffer (PBS comprising 0.2% BSA and 0.05% Tween 20) was mixed together with anti-GH mouse mAb diluted with the same buffer to the final concentration of 30 ng/mL, and the mixture was added at 50 µL/well, and the plate was left still at room temperature for 2 hours. The plate was washed with washing buffer (PBS comprising 0.05% Tween 20), followed by adding, at 50 µL/well, HRP-labeled goat anti-mouse IgG antibody (Jackson ImmunoResearch Laboratories Inc.) diluted with assay buffer to 0.1 µg/mL, and the plate was left still at room temperature for 2 hours. The plate was further washed with washing buffer, followed by adding TMB solution as a substrate of HRP at 50 µL/well, and allowed to react with the enzyme at room temperature. Ten minutes later, the reaction was stopped by adding 0.5 mol/L H₂SO₄, and then the absorbance at 450 nm was measured with a plate reader. IC₂₀ (nmol/L) values of the anti-GH mouse mAb was determined by concentration-dependent curves of human GH and GH-analogous protein to calculate the cross-reactivities by the following formula 2. Cross-reactivity to GH-analogous protein (%) = IC20 (human GH) / IC20 (GH-analogous protein) x 100

### <Example 5: Production of Anti-GH Mouse mAb>

### (1) Production of Anti-GH Mouse mAb

Female mice (7-week-old) were subcutaneously immunized with 5 µg or 10 µg of recombinant human GH (FUJIFILM Wako Pure Chemical Corporation, Pharmaceutical and Medical Device Regulatory Science Society of Japan) along with various adjuvants repeatedly twice a week for 7 to 13 times. Blood was collected from these mice over time, and the plasma antibody titers were measured with ELISA described in Example 1. Ten µg of recombinant human GH was finally immunized to a tail vein or intraperitoneal of the mice whose plasma antibody titer was satisfactory increased, and the mice were euthanized 3 to 4 days later to resect their lymph nodes and spleens. The isolated lymphocytes and mouse myeloma cell line P3U1 (JCRB0708, National Institutes of Biomedical Innovation, Health and Nutrition) were electrically fused and cultured in a semisolid selective medium comprising hypoxanthine-aminopterin-thymidine (HAT) (STEMCELL Technologies) for 8 to 12 days in a CO₂ incubator. Proliferated monoclonal hybridoma was picked up by a colony picker to transfer onto a 96-well culture plate with a liquid medium comprising hypoxanthine-thymidine (HT) (STEMCELL Technologies) for culture in a CO₂ incubator for 3 to 4 days.

Ten-fold dilution solution of the hybridoma culture supernatant thus obtained was subjected to ELISA described in Example 1, and the GH binding activity was measured. Two-fold dilution solution of the culture supernatant was subjected to GH-GHR binding inhibition assay in accordance with the method described in Example 2(a), and the neutralizing activity against GH was measured. The anti-GH mouse mAb-producing hybridoma that has shown 70% or more of inhibitory activity on the GH-GHR binding inhibition assay was expanded and cultured in a 24-well plate, and the hybridoma which culture supernatant reproduced the GH binding activity and GH-GHR binding inhibitory activity and produced antibodies with a single IgG subtype was selectively obtained as a neutralizing mAb-producing hybridoma. They were cryopreserved.

The anti-GH mouse mAb-producing hybridoma selectively obtained above was expanded and cultured in a DMEM medium comprising 10% Ultra Law IgG, and the culture supernatant was applied to purification with rProtein A Sepharose FF (GE Healthcare Inc.) to obtain anti-GH mouse mAb. In addition, the purity was confirmed with SDS-PAGE, and the antibody concentration was quantified with the absorbance at 280 nm using molecular extinction coefficient (1 mg/mL) of 1.38.

The mouse mAb sample thus prepared was examined on the GH-GHR binding inhibition assay and GH-dependent Nb₂ cell proliferation inhibition assay described in Example 2, the cross-reactivity to experimental animal-derived GHs described in Example 3, and the cross-reactivity to GH-analogous proteins described in Example 4, and then the anti-GH mouse mAb, 13H02 (IgG2a/**κ**) which showed outstanding overall feature in terms of the GH binding properties and GH neutralizing activity was selectively obatined.

### (2) Profile of Anti-GH Mouse mAb, 13H02

Regarding the anti-GH mouse mAb (13H02), the GH-GHR binding inhibitory activity (IC₅₀) and GH-dependent Nb₂ cell proliferation inhibitory activity (IC₅₀) measured by the method in Example 2 are shown in Table 1, the cross-reactivity to experimental animal-derived GHs measured by the method in Example 3 is shown in Table 2, and the cross-reactivity to GH-analogous proteins measured by the method in Example 4 is shown in Table 3, respectively. When a commercially available anti-GH mouse mAb (MAB1067) was used as a positive control, the results thereof are also shown.

**[Table 1]**

| **GH-GHR binding inhibitory activity (IC₅₀) and GH-dependent Nb₂ cell proliferation inhibitory activity (IC₅₀)** | | |
|---|---|---|
| | **GH-GHR binding inhibitory activity (pmol/L)** | **GH-dependent Nb₂ cell proliferation inhibitory activity (pmol/L)** |
| 13H02 | 65.3 | 3.36 |
| MAB1067 | 6,860 | 21,500 |

**[Table 2]**

| **Cross-reactivity to experimental animal-derived GHs** | | | |
|---|---|---|---|
| | **Mouse GH** | **Rat GH** | **Monkey GH** |
| 13H02 | < 1% | < 1% | 41% |

**[Table 3]**

| **Cross-activity to GH-analogous proteins** | | |
|---|---|---|
| | PL | PRL |
| 13H02 | < 1% | < 1% |

The 13H02 strongly inhibited the GH activity with IC₅₀ = 65.3 pmol/L in the GH-GH GHR binding assay, and with IC₅₀ = 3.36 pmol/L in the GH-dependent Nb₂ cell proliferation assay. The 13H02 significantly bound to monkey GH with the cross-reactivity of 41% although it did not substantially bind to mouse GH and rat GH as the cross-reactivities to them were <1%. Further, 13H02 did not substantially bind to the GH-analogous proteins (PL and PRL) as the cross-reactivities to them were <1%.

### <Example 6: Efficacy of Anti-GH Mouse mAb (13H02) on GH-Supplemented Hypophysectomized Rats>

The efficacy of the anti-GH mouse mAb (13H02) selectively obtained in Example 5 was evaluated on GH-supplemented hypophysectomized rats imitating acromegaly.

A 2-week type ALZET osmotic pump (Muromachi Kikai Co., Ltd.) filled with recombinant human GH (30 µg/day; Sandoz AG)was subcutaneously implanted to hypophysectomized SD rats (6-week-old; Japan SLC, Inc.) in which the pituitary gland was resected at the age of 4-week and which exhibited reduced serum IGF-1 due to the endogenous GH deficiency (hereinafter, also referred to as "hypophysectomized rat"). The 13H02 was subcutaneously administered to the GH-supplemented hypophysectomized rat one day after starting GH release at a single dose of 0.01, 0.1, 1, 5, and 10 mg/kg (n=5 or 6). The normal control group (Normal) was hypophysectomized rat without GH-supplementation (hypophysectomized rat without recombinant human GH supplmentation); whereas as the negative control group, control mouse IgG1 (M1411; Leinco Technologies, Inc.) was administered to the GH-supplemented hypophysectomized rat. The serum IGF-1 values at 2 days after administration of 13H02 (average values and standard errors) are shown in Figure 1. The 13H02 significantly reduced the serum IGF-1 values in the administration group of 1, 5, and 10 mg/kg (###: p<0.001 vs normal control group/Aspin-Welch t-test, *: p<0.05 vs disease control group/Steel's multiple comparison test).

### <Examples 7: Determination of Variable Region Amino Acid Sequence of 13H02>

From the anti-GH mouse mAb (13H02)-producing hybridoma selected in Example 5, mRNA was prepared using Dynabeads mRNA DIRECT Kit (Thermo Fischer Scientific Inc.) according to the instruction attached in the kit. cDNA was synthesized with SMARTer RACE 5'/3' Kit (Takara Bio Inc.) using 5 µL of the mRNA solution as a template. The variable region genes (VH and VL) of the antibody H chain and L chain were amplified from the cDNA by PCR with KOD-Fx Neo (TOYOBO Co., Ltd.) as polymerase using 10× Universal Primer A Mix provided in the kit as a forward primer, and 5'-RACE Reverse primer designed from the known mouse antibody constant region sequence as a reverse primer.

The amplified PCR products were integrated into a cloning vector, pCR4Blunt-TOPO (Thermo Fisher Scientific Inc.), followed by transfecting that into Stellar competent cells (Takara Bio Inc.) to obtain a transformant. The plasmid was prepared from the clones which were confirmed to have VH and VL inserts by the colony PCR with SapphireAmp Fast PCR Master Mix (Takara Bio Inc.), and the amino acid sequence was estimated from the determined DNA sequence of variable regions. CDR1 to 3 of these H chain and L chain in the variable region amino acid sequence were determined according to the method of Kabat et al., using Discovery Studio (Biovia). The DNA sequence and amino acid sequence of the H chain variable region (VH) of 13H02 are set forth in SEQ ID NO: 1 and SEQ ID NO: 19, respectively, and the DNA sequence and amino acid sequence of the L chain variable region (VL) are set forth in SEQ ID NO: 2 and SEQ ID NO: 20, respectively. The amino acid sequence of the mature 13H02_VH in which a signal peptide is removed, that starts from position 20 of SEQ ID NO: 19 is set forth in SEQ ID NO: 3, and the amino acid sequence of the mature 13H02_VL in which a signal peptide is removed, that starts from position 23 of SEQ ID NO: 20 is set forth in SEQ ID NO: 4. The VH complementarity-determining region (CDR) 1, 2, and 3 of 13H02 were SSEIS (SEQ ID NO: 5), WIYPGTGSSKFNQKFTG (SEQ ID NO: 6), and RGFFGGSFDY (SEQ ID NO: 7), respectively. The VLCDR1, 2, and 3 of 13H02 were TATSSVSSSYLH (SEQ ID NO: 8), STSNLAS (SEQ ID NO: 9), and HQYHHSPPT (SEQ ID NO: 10), respectively.

### <Example 8: Preparation of Anti-GH Mouse-Human Chimeric mAb>

The H chain and L chain constant region genes of human IgG1/**κ** antibody were inserted into pcDNA3.4 (Thermo Fisher Scientific Inc.) separately to construct expression cassette vectors for mouse-human chimeric antibody H chain and L chain, respectively (hereinafter, expression cassette vectors for mouse-human chimeric antibody H chain and mouse-human chimeric antibody L chain, comprising constant region genes of human IgG1/**κ** antibody are referred to as "cassette vectors for human IgG1/**κ** mouse-human chimeric antibody (H chain, L chain)"). The VH and VL genes of 13H02 isolated in Example 7 were inserted into the cassette vector for human IgG1/**κ** mouse-human chimeric antibody (H chain, L chain) to construct expression vector for recombinant mouse-human chimeric antibody (H chain , L chain), respectively. The expression vectors for H chain and L chain of recombinant mouse-human chimeric antibody were co-transfected into ExpiCHO-S cell line with ExpiFectamine CHO Transfection Kit (Thermo Fisher Scientific Inc.) at H chain:L chain = 1:1 (weight ratio), and the transfected cells were cultured with shaking for 8 days in a CO₂ incubator shaker (37°C, 8% CO₂). The culture supernatant was collected by centrifugation, and the antibody was purified with rProtein A Sepharose FF (GE Healthcare Inc.) to obtain recombinant anti-GH mouse-human chimeric mAb (Ch-13H02). The purity was confirmed with SDS-PAGE, and the antibody concentration was quantified with the absorbance at 280 nm as molecular extinction coefficient (1 mg/mL) of 1.38.

### <Example 9: GH-Dependent Nb₂ Proliferation Inhibitory Activity of Anti-GH Mouse-Human Chimeric mAb (Ch-13H02)>

The GH-neutralizing activity of the recombinant anti-GH mouse-human chimeric mAb (Ch-13H02) prepared in Example 8 was measured by the GH-dependent Nb₂ cell proliferation inhibition assay described in Example 2(b). The results are shown in Table 4.

**[Table 4]**

| **GH-dependent Nb₂ cell proliferation inhibition assay (IC₅₀)** | |
|---|---|
| | **GH-dependent Nb₂ cell proliferation inhibitory activity (pmol/L)** |
| Ch-13H02 | 6.27 |

It was confirmed that Ch-13H2 has the GH-dependent Nb₂ cell proliferation inhibitory activity with IC₅₀ = 6.27 pmol/L, which is approximately equal to that of hybridoma-derived parent mouse antibody (13H02), and that H chain and L chain genes that contribute to the activity were isolated from 13H02-producing hybridoma.

### <Example 10: Humanization of Anti-GH Mouse mAb (13H02)>

Design for humanized VH and VL of 13H02 was performed according to the report of Queen C. et al. (Proc. Natl. Acad. Sci. USA 86, 10029-10033, 1989) and the method of Tsurushita N. et al. (Methods 36, 69-83, 2005). Briefly stated, it is as follows. First, 3-dimensional molecular models for VH and VL of 13H02 were constructed using the original algorithm of JN Biosciences LLC, and amino acid residues in the framework region that are important to keep the structure of CDRs were identified. In addition, the human VH and VL amino acid sequences which are the most homologous with the VH and VL of the present antibody were identified, respectively from publicly available database. Three each of the CDR sequences of VH and VL of 13H02 were grafted along with the amino acid residues important to keep the CDR structure in the framework region to the human antibody framework region sequences identified above, and thereby humanized 13H02 (Hu-13H02) was designed.

The amino acid sequence of the humanized Hu-13H02_VH is set forth in SEQ ID NO: 11, and the amino acid sequence of the mature Hu-13H02_VH in which a signal peptide is removed, that starts from position 20 of SEQ ID NO: 11 is set forth in SEQ ID NO: 12. The amino acid sequence of Hu-13H02_VL is set forth in SEQ ID NO: 13, and the amino acid sequence of the mature Hu-13H02_VL in which a signal peptide is removed, that starts from position 23 of SEQ ID NO: 13 is set forth in SEQ ID NO: 14.

### <Example 11: Preparation of Anti-GH Humanized Antibody>

DNA (SEQ ID NO: 15) encoding the VH amino acid sequence (SEQ ID NO: 11) and DNA (SEQ ID NO: 16) encoding the VL amino acid sequence (SEQ ID NO: 13) of the anti-GH humanized antibody (Hu-13H02) designed in Example 10 were inserted into cassette vectors for the human IgG1/**κ** mouse-human chimeric antibody (H chain, L chain) constructed in Example 8 to construct expression vectors for recombinant humanized antibody (H chain, L chain) (hereinafter, also referred to as "expression vectors for Hu-13H02 (H chain, L chain)"), respectively.

For the purpose of improving blood kinetics, a Fc mutant(Hu-13H02m) in which Met⁴²⁸ was substituted with a Leu residue in the H chain of Hu-13H02 was prepared. Specifically, DNA encoding VH amino acid sequence of the anti-GH humanized antibody (Hu-13H02) was inserted into the expression cassette vector for H chain in which the gene encoding the human IgG1 constant region where Met⁴²⁸ has been substituted with Leu⁴²⁸ was inserted into pcDNA3.4. (hereinafter, also referred to "expression vectors for Hu-13H02m (H chain and L chain)").

The expression vectors for Hu-13H02 or Hu-13H02m (H chain and L chain) were each co-transfected into ExpiCHO-S cell line with ExpiFectamine CHO Transfection Kit (Thermo Fisher Scientific Inc.) at H chain:L chain = 1:1 (weight ratio), and the transfected cells were cultured with shaking according to Standard protocol or Max titer protocol of the expression system. The culture supernatant was collected by centrifugation, and the antibody was purified with HiTrap rProtein A FF Column (GE Healthcare Inc.) to obtain a sample of Hu-13H02 or Hu-13H02m. The purity was confirmed with SDS-PAGE, and the antibody concentration was quantified with the absorbance at 280 nm as molecular extinction coefficient (1 mg/mL) of 1.38. The content of endotoxin of the samples to be used for a rat efficacy test was measured using Endospecy ES-50M (SEIKAGAKU CORPORATION), and was confirmed to be <1 EU/mg. The sample of Hu-13H02 or Hu-13H02m thus prepared was used for subsequent tests. The amino acid sequence in the heavy chain constant region of Hu-13H02m is set forth in SEQ ID NO: 17, and the amino acid sequence in the light chain constant region of Hu-13H02m is set forth in SEQ ID NO: 18.

### <Example 12: GH-Dependent Nb₂ Proliferation Inhibitory Activities of Anti-GH Chimeric Antibody and Anti-GH Humanized Antibodies>

The GH-dependent Nb₂ cell proliferation inhibition assay described in Example 2(b) was performed on the recombinant anti-GH humanized antibody (Hu-13H02) and the Fc mutant of the anti-GH humanized antibody (Hu-13H02m) prepared in Example 11, and the chimeric antibody (Ch-13H02) prepared in Example 8 to determine IC₅₀ against human GH from the concentration-dependent curves. In addition, the IC₅₀ values for the monkey GH was determined using the monkey GH prepared in Example 3 instead of the human GH in Example 2(b). The human GH at 9.1 pmol/L and the monkey GH at 41 pmol/L as a final concentration was added, respectively. The results are shown in Table 5.

**[Table 5]**

| **Neutralizing activity of anti-GH chimeric antibody and anti-GH humanized antibody in GH-dependent Nbz cell proliferation inhibition assay (IC₅₀)** | | |
|---|---|---|
| **Name of antibody clone** | **Human-GH** (pmol/L) | **Monkey-GH** (pmol/L) |
| Ch-13H02 | 2.65 | 13.6 |
| Hu-13H02 | 16.5 | 48.0 |
| Hu-13H02m | 14.7 | 30.1 |

Hu-13H02 and Hu-13H02m showed strong GH-dependent Nb₂ cell proliferation inhibitory activities to human GH and monkey GH, the same as Ch-13H02.

### <Example 13: Binding Properties of Anti-GH Humanized Antibodies to Human GH and Monkey GH>

The binding properties of the recombinant anti-GH humanized antibody (Hu-13H02) and the Fc mutant thereof (Hu-13H02m) prepared in Example 11, and the chimeric antibody (Ch-13H02) prepared in Example 8 to the human GH (Pharmaceutical and Medical Device Regulatory Science Society of Japan) and the monkey GH (Example 3) were measured with SPR using BIACORE 8K+ (GE Healthcare Inc.). The antibody samples (1 µg/mL) of the three antibodies (Hu-13H02, Hu-13H02m, and Ch-13H02) were captured by anti-human IgG (Fc) antibody immobilized on a CM5 sensor chip, respectively. Next, the human GH ranging from 0.13 to 8.0 nmol/L, or the monkey GH ranging from 0.50 to 32 nmol/L was added to calculate binding and dissociation parameters. The results are shown in Table 6.

**[Table 6]**

| **Binding properties of anti-GH chimeric antibody and anti-GH humanized antibody to human GH and monkey GH** | | | | | | |
|---|---|---|---|---|---|---|
| **Name of antibody clone** | **Human GH** | | | **Monkey GH** | | |
| | kₒₙ (L/mol • s) | k_{off} (1/s) | K_{D} (mol/L) | kₒₙ (L/mol • s) | k_{off} (1/s) | K_{D} (mol/L) |
| Ch-13H02 | 1.9 × 10⁶ | 2.4 × 10⁻⁵ | 1.2 × 11⁻¹¹ | 4.5 × 10⁵ | 3.4 × 10⁻⁵ | 7.4 × 10⁻¹¹ |
| Hu-13H02 | 2.0 × 10⁶ | 3.8 × 10⁻⁵ | 1.9 × 10⁻¹¹ | 4.7 × 10⁵ | 6.2 × 10⁻⁵ | 1.3 × 10⁻¹⁰ |
| Hu-13H02m | 2.1 × 10⁶ | 3.8 × 10⁻⁵ | 1.9 × 10⁻¹¹ | 5.2 × 10⁵ | 6.9 × 10⁻⁵ | 1.3 × 10⁻¹⁰ |

Hu-13H02 and Hu-13H02m retained human GH-binding activities which were approximately equivalent to Ch-13H02, and exhibited strong binding activities to the monkey GH as well.

### <Example 14: Cross-Reactivity of Anti-GH Chimeric Antibody and Anti-GH Humanized Antibodies to GH-Analogous Proteins>

The cross-reactivities of the recombinant anti-GH humanized antibody (Hu-13H02) and the Fc variant thereof (Hu-13H02m) prepared in Example 11, and the parent chimeric antibody (Ch-13H02) prepared in Example 8 to proteins (placental lactogen (PL) and prolactin (PRL)) which exhibit structural similarity to GH were measured in a competitive ELISA similar to that described in Example 4. IC₅₀ (nmol/L) for each of the anti-GH antibodies was determined by concentration-dependent curves of human GH and GH-analogous protein. The cross-reactivity to the GH-analogous protein was calculated by the following formula 3. Cross-reactivity th GH-analogous protein (%) = IC50 (human GH) / IC50 (GH-analogous protein) x 100

The results are shown in Table 7.

**[Table 7]**

| **Cross-activitv to GH-analogous proteins** | | |
|---|---|---|
| | PL | PRL |
| Ch-13H02 | < 1% | < 1 % |
| Hu-13H02 | < 1% | < 1% |
| Hu-13H02m | < 1% | < 1% |

Ch-13H02, Hu-13H02, and Hu-13H02m did not substantially bind to the GH-analogous proteins as any of the cross-reactivities to PL and PRL were <1%.

### <Example 15: Efficacy of Anti-GH Mouse-Human Chimeric mAb (Ch-13H02) and Anti-GH Humanized Antibody Fc Mutant (Hu-13H02m) on GH-Supplemented Hypophysectomized Rats>

The efficacy of the anti-GH mouse-human chimeric mAb (Ch-13H02) and anti-GH humanized antibody Fc mutant (Hu-13H02m) was evaluated on the GH-supplemented hypophysectomized rats described in Example 6.

Hypophysectomized SD rats (6-week-old; Japan SLC, Inc.) in which the pituitary gland was resected at the age of 4-week and which exhibited reduced serum IGF-1 due to endogenous GH deficiency was subcutaneously implanted with a 3-day type ALZET osmotic pump (Muromachi Kikai Co., Ltd.) filled with recombinant human GH (30 µg/day; Sandoz AG). The anti-GH antibodies (Ch-13H02 and Hu-13H02m) were subcutaneously administered to the GH-supplemented hypophysectomized rats one day after starting release of GH at a single dose of 1, 3, and 10 mg/kg (n=6). The normal control group (Normal) is hypophysectomized rat without GH-supplementation (hypophysectomized rat without recombinant human GH-supplementation); whereas as the negative control group, control human IgG1 (BE0297; Bio X Cell, Inc.) was administered to the GH-supplemented hypophysectomized rat. The serum IGF-1 values 2 days after administration of Ch-13H02 are shown in Figure 2, and the serum IGF-1 values at 2 days after administration of Hu-13H02m (average values and standard errors) are shown in Figure 3. Ch-13H02 significantly reduced the serum IGF-1 values in the administration group of 3 and 10 mg/kg, and Hu-13H02m significantly reduced the serum IGF-1 values in the administration group of 3 and 10 mg/kg (###: p<0.001 vs normal control group/Aspin-Welch t-test, *: p<0.05 vs negative control group /Steel's multiple comparison test).

The sequences described herein are shown in the following.
<1; DNA; Mus musculus> 
ATGGAATGGAACTGGGTCGTTCTCTTCCTCCTGTCATTAACTGCAGGTGTCTATGCCCAGGGTCAGATGCAGCAGTCTGG
AGGTGAGTTGGTGAATCCTGGGTCTTCAGTGAAGATGTCCTGCAAGACTTCTGGCTTCACCTTCACCAGTAGTGAAATAA
GTTGGTTGAAGCAGAAGCCTGGACAGAGTCTTGAGTGGATTGCATGGATTTATCCTGGAACTGGTAGTAGTAAGTTTAAT
CAGAAGTTCACGGGCAAGGCCCAACTGACTGCAGAAACATCCTCCAGCACAGCCTACATGCAACTCAGCAGCCTGACATC
TGAGGACTCTGCCATCTATTACTGTGCAAGACGGGGGTTCTTCGGTGGTAGTTTTGACTACTGGGGCCAAGGCACCACTC
TCACAATCTCCTCA
<2; DNA; Mus musculus> 
ATGGATTTTCAGGTGCAGATTTTCAGCTTCCTGCTAATCAGTGCCTCAGTCGTAATGTCCAGAGGACAAATTGTTCTCGC
CCAGTCTCCAGCAATCATGTCTGCATCTCTAGGGGAACGGGTCACCATGACCTGCACTGCCACCTCAAGTGTAAGTTCCA
GTTACTTGCACTGGTACCAGCAGAAGCCAGGATCCTCCCCCAAACTCTGGATTTATAGCACATCCAACCTGGCTTCTGGA
GTCCCAGCTCGCTTCAGTGGCAGTGGGTCTGGGACCTCTTACTCTCTCACAATCAGCAGCATGGAGGCTGAAGATGCTGC
CACTTATTACTGCCACCAGTATCATCATTCCCCACCCACGTTCGGTGCTGGGACCAAGCTGGAGCTGAAA
<3; PRT1; Mus musculus> 
QGQMQQSGGELVNPGSSVKMSCKTSGFTFTSSEISWLKQKPGQSLEWIAWIYPGTGSSKFNQKFTGKAQLTAETSSSTAY
MQLSSLTSEDSAIYYCARRGFFGGSFDYWGQGTTLTISS
<4; PRT1; Mus musculus> 
QIVLAQSPAIMSASLGERVTMTCTATSSVSSSYLHWYQQKPGSSPKLWIYSTSNLASGVPARFSGSGSGTSYSLTISSME
AEDAATYYCHQYHHSPPTFGAGTKLELK
<5; PRT1; Mus musculus>
   SSEIS
<6; PRT1; Mus musculus>
   WIYPGTGSSKFNQKFTG
<7: PRT1; Mus musculus>
   RGFFGGSFDY
<8; PRT1; Mus musculus>
   TATSSVSSSYLH
<9; PRT1; Mus musculus>
   STSNLAS
<10; PRT1; Mus musculus>
   HQYHHSPPT
<11; PRT1; Artificial> 
MEWNWVVLFLLSLTAGVYAQVQMVQSGAEVKKPGASVKVSCKASGFTFTSSEISWLRQAPGQRLEWIAWIYPGTGSSKFN
QKFTGKATLTADTSASTAYMELSSLRSEDTAVYYCARRGFFGGSFDYWGQGTTVTVSS
<12; PRT1: Artificial> 
QVQMVQSGAEVKKPGASVKVSCKASGFTFTSSEISWLRQAPGQRLEWIAWIYPGTGSSKFNQKFTGKATLTADTSASTAY
MELSSLRSEDTAVYYCARRGFFGGSFDYWGQGTTVTVSS
<13; PRT1; Artificial> 
MDFQVQIFSFLLISASVVMSRGDIQLTQSPSFLSASVGDRVTITCTATSSVSSSYLHWYQQKPGKAPKLWIYSTSNLASG
VPSRFSGSGSGTEFTLTISSLQPEDFATYYCHQYHHSPPTFGGGTKVEIK
<14; PRT1; Artificial> 
DIQLTQSPSFLSASVGDRVTITCTATSSVSSSYLHWYQQKPGKAPKLWIYSTSNLASGVPSRFSGSGSGTEFTLTISSLQ
PEDFATYYCHQYHHSPPTFGGGTKVEIK
<15; DNA; Artificial> 
ATGGAATGGAACTGGGTCGTGCTGTTCCTGCTGTCTCTGACCGCTGGCGTGTACGCTCAGGTTCAGATGGTTCAGTCTGG
CGCCGAAGTGAAGAAACCTGGCGCCTCTGTGAAGGTGTCCTGCAAGGCTTCCGGCTTCACCTTTACCTCCAGCGAGATCT
CTTGGCTGAGACAGGCTCCTGGCCAGCGGCTGGAATGGATCGCTTGGATCTATCCTGGCACCGGCTCCTCCAAGTTCAAC
CAGAAATTCACCGGCAAAGCCACCCTGACCGCCGACACCTCTGCTTCTACCGCCTACATGGAACTGTCCAGCCTGAGATC
TGAGGACACCGCCGTGTACTACTGCGCCAGAAGAGGCTTCTTCGGCGGCTCCTTTGATTACTGGGGCCAGGGCACAACCG
TGACCGTTTCTTCT
<16; DNA; Artificial> 
ATGGACTTCCAGGTGCAGATCTTCTCCTTCCTGCTGATCTCCGCCTCCGTGGTCATGTCCAGAGGCGACATTCAGCTG
ACCCAGTCTCCATCCTTCCTGTCCGCCTCTGTGGGCGACAGAGTGACCATTACCTGTACCGCTACCTCCTCCGTGTCC
TCCTCTTACCTGCACTGGTATCAGCAGAAGCCCGGCAAGGCTCCCAAGCTGTGGATCTACTCCACCTCCAATCTGGCC
TCTGGCGTGCCCTCTAGATTCTCCGGATCTGGCTCTGGAACCGAGTTTACCCTGACAATCTCCAGCCTGCAGCCTGAG
GACTTCGCCACCTACTACTGCCACCAGTACCACCTCTCCACCTACCTTTGGCGGAGGCACCAAGGTGGAAATCAAG
<17; PRT1; Artificial> 
ASTKGPSVFPLAPSSKSTSGGTAALGCLVKDYFPEPVTVSWNSGALTSGVHTFPAVLQSSGLYSLSSVVTVPSSSLGTQT
YICNVNHKPSNTKVDKKVEPKSCDKTHTCPPCPAPELLGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAPIEKTISKAKGQPREPQVYTLPPSRDE
LTKNQVSLTCLVKGFYPSDIAVEWESNGQPENNYKTTPPVLDSDGSFFLYSKLTVDKSRWQQGNVFSCSVLHEALHNHYT
QKSLSLSPGK
<18; PRT1; Homo sapiens>
<19; PRT1; Mus musculus> 
MEWNWVVLFLLSLTAGVYAQGQMQQSGGELVNPGSSVKMSCKTSGFTFTSSEISWLKQKPGQSLEWIAWIYPGTGSSKFN
QKFTGKAQLTAETSSSTAYMQLSSLTSEDSAIYYCARRGFFGGSFDYWGQGTTLTISS
<20; PRT1; Mus musculus>

MDFQVQIFSFLLISASVVMSRGQIVLAQSPAIMSASLGERVTMTCTATSSVSSSYLHWYQQKPGSSPKLWIYSTSNLASG
VPARFSGSGSGTSYSLTISSMEAEDAATYYCHQYHHSPPTFGAGTKLELK

## Claims

1. An antibody specifically binding to human growth hormone or an antigen-binding fragment thereof, comprising a heavy chain variable region (VH) and a light chain variable region (VL), wherein
the VH comprises a VH complementarity-determining region (CDR) 1 (VHCDR1), a VHCDR2, and a VHCDR3,
the VHCDR1 comprises an amino acid sequence set forth in SEQ ID NO: 5, or an amino acid sequence having substitution, addition, or deletion of one or two amino acids in the amino acid sequence set forth in SEQ ID NO: 5,
the VHCDR2 comprises an amino acid sequence set forth in SEQ ID NO: 6, or an amino acid sequence having substitution, addition, or deletion of one or two amino acids in the amino acid sequence set forth in SEQ ID NO: 6,
the VHCDR3 comprises an amino acid sequence set forth in SEQ ID NO: 7, or an amino acid sequence having substitution, addition, or deletion of one or two amino acids in the amino acid sequence set forth in SEQ ID NO: 7,
the VL comprises a VL complementarity-determining region (CDR) 1 (VLCDR1), a VLCDR2, and a VLCDR3,
the VLCDR1 comprises an amino acid sequence set forth in SEQ ID NO: 8, or an amino acid sequence having substitution, addition, or deletion of one or two amino acids in the amino acid sequence set forth in SEQ ID NO: 8,
the VLCDR2 comprises an amino acid sequence set forth in SEQ ID NO: 9, or an amino acid sequence having substitution, addition, or deletion of one or two amino acids in the amino acid sequence set forth in SEQ ID NO: 9, and
the VLCDR3 comprises an amino acid sequence set forth in SEQ ID NO: 10, or an amino acid sequence having substitution, addition, or deletion of one or two amino acids in the amino acid sequence set forth in SEQ ID NO: 10,

2. An antibody specifically binding to human growth hormone or an antigen-binding fragment thereof, comprising a heavy chain variable region (VH) and a light chain variable region (VL), wherein
the VH comprises an amino acid sequence set forth in SEQ ID NO: 5 as a VH complementarity-determining region (CDR) 1 (VHCDR1), an amino acid sequence set forth in SEQ ID NO: 6 as a VHCDR2, and an amino acid sequence set forth in SEQ ID NO: 7 as a VHCDR3, and
the VL comprises an amino acid sequence set forth in SEQ ID NO: 8 as a VL complementarity-determining region (CDR) 1 (VLCDR1), an amino acid sequence set forth in SEQ ID NO: 9 as a VLCDR2, and an amino acid sequence set forth in SEQ ID NO: 10 as a VLCDR3.

3. The antibody or antigen-binding fragment thereof according to claim 1 or 2, wherein the antibody or antigen-binding fragment thereof binds to human growth hormone at an equilibrium dissociation constant (K_{D}) of 1.0 × 10⁻⁸ mol/L or less as measured by a surface plasmon resonance method.

4. The antibody or antigen-binding fragment thereof according to any one of claims 1 to 3, wherein the antibody or antigen-binding fragment thereof neutralizes action of growth hormone.

5. The antibody or antigen-binding fragment thereof according to any one of claims 1 to 4, comprising:
the VH comprising an amino acid sequence having substitution, addition, or deletion of one or several amino acids in regions other than the CDR1 to CDR3 in an amino acid sequence set forth in SEQ ID NO: 3, 11, 12, or 19, or an amino acid sequence having an identity of 90% or more with the amino acid sequence set forth in SEQ ID NO: 3, 11, 12, or 19, and/or
the VL comprising an amino acid sequence having substitution, addition, or deletion of one or several amino acids in regions other than the CDR1 to CDR3 in an amino acid sequence set forth in SEQ ID NO: 4, 13, 14, or 20, or an amino acid sequence having an identity of 90% or more with the amino acid sequence set forth in SEQ ID NO: 4, 13, 14, or 20.

6. The antibody or antigen-binding fragment thereof according to claim 5, comprising the VH having the amino acid sequence set forth in SEQ ID NO: 3, 11, 12, or 19, and/or the VL having the amino acid sequence set forth in SEQ ID NO: 4, 13, 14, or 20.

7. The antibody or antigen-binding fragment thereof according to any one of claims 1 to 6, wherein the antibody is a monoclonal antibody.

8. The antibody or antigen-binding fragment thereof according to any one of claims 1 to 7, wherein the antibody is a chimeric antibody, humanized antibody, veneered antibody, or fully human antibody.

9. The antibody or antigen-binding fragment thereof according to any one of claims 1 to 8, wherein the antibody comprises:
a heavy chain constant region comprising an amino acid sequence having substitution, addition, or deletion of one or several amino acids in an amino acid sequence set forth in SEQ ID NO: 17, or a heavy chain constant region comprising an amino acid sequence having an identity of 90% or more with the amino acid sequence set forth in SEQ ID NO: 17, and/or
a light chain constant region comprising an amino acid sequence having substitution, addition, or deletion of one or several amino acids in an amino acid sequence set forth in SEQ ID NO: 18, or a light chain constant region comprising an amino acid sequence having an identity of 90% or more with the amino acid sequence set forth in SEQ ID NO: 18.

10. The antibody or antigen-binding fragment thereof according to any one of claims 1 to 9, wherein the antibody comprises the heavy chain constant region having the amino acid sequence set forth in SEQ ID NO: 17, and/or the light chain constant region having the amino acid sequence set forth in SEQ ID NO: 18.

11. The antibody or antigen-binding fragment thereof according to any one of claims 1 to 10, wherein the antigen-binding fragment is Fab, Fab', F(ab')2, Fv, or scFv.

12. A nucleic acid encoding the antibody or antigen-binding fragment thereof according to any one of claims 1 to 11.

13. A vector comprising the nucleic acid according to claim 12.

14. A cell comprising the nucleic acid according to claim 12 or the vector according to claim 13.

15. A medicine comprising the antibody or antigen-binding fragment thereof according to any one of claims 1 to 11.

16. The medicine according to claim 15, for decreasing the level of insulin-like growth factor 1 (IGF-1) in the blood.

17. The medicine according to claim 15 or 16, for treating and/or preventing a disease selected from the group consisting of acromegaly, gigantism, cancer, diabetic nephropathy, arthritis, and pneumonia.

18. A method for decreasing the level of an insulin-like growth factor 1 (IGF-1) in the blood, comprising administration of a therapeutically effective amount of the antibody or antigen-binding fragment thereof according to any one of claims 1 to 11.
